# EUROPEAN PATENT APPLICATION

(11) **EP 4 124 348 A1**
(43) Date of publication of application: **01.02.2023**
(21) Application number: 21382724.9
(22) Date of filing: 30.07.2021
(51) Int. Cl.: A61K 49/00, A61K 47/50, A61K 47/69

(54) **NANOPARTICLES FOR CELL DELIVERY**

(71) Applicant: 4basebio UK Ltd, Cambridge CB24 5QE (GB); 4basebio, S.L.U., 28049 Cantoblanco (Madrid) (ES)
(72) Inventor: Walker, Amy, Over, Cambridge, CB24 5QE (GB); Lanckriet, Heikki, Over, Cambridge, CB24 5QE (GB); Picher, Angel, 28049 Cantoblanco (Madrid) (ES)
(74) Representative: Boult Wade Tennant LLP

(57) **Abstract**

Nanoparticles suitable for delivery of a linear DNA molecule (e.g. a closed linear DNA molecule) to a target cell are provided. Further provided are uses of the nanoparticles including the use of the nanoparticles for treating disease.

## Description

### TECHNICAL FIELD

The present invention relates to nanoparticles suitable for delivery of a linear DNA molecule (e.g. a closed linear DNA molecule) to a target cell. The present invention also relates to uses of the nanoparticles for example uses of the nanoparticles in treating a disease.

### BACKGROUND

Gene delivery for therapy or other purposes is well-known, particularly for the treatment of singlegenetic disorders such as cystic fibrosis.

Gene delivery systems fall into three broad classes: those that involve direct injection of naked DNA; those that make use of viruses or genetically modified viruses to deliver the DNA; and those that make use of non-viral delivery agents.

Although viruses as delivery agents have the advantages of high efficiency and high cell selectivity, they have the disadvantages of toxicity, risk of insertional mutagenesis, production of inflammatory responses, high likelihood of eliciting a host immune response and limited packaging capacity.

Non-viral gene delivery systems are based on the compaction of genetic material into nanometric particles by electrostatic interaction between the negatively charged phosphate backbone of DNA and cationic lipids, peptides or other polymers (Erbacher, P. et al, Gene Therapy, 1999, 6, 138-145). Known complexes for delivery include lipoplex for lipid based nucleic acid complexes, polyplex for peptide or polymer-based complexes and lipopolyplex for hybrid systems (Feigner et al., Human Gene Therapy 8, 1997, 511-512).

Non-viral lipid vector formulations which complex nucleic acid with cationic lipids suffer from problems of poor tissue penetration, non-specific charge-mediated binding to cells, and interactions with serum proteins which can lead to inflammatory responses. Using an anionic lipid as an alternative is appealing because it offers the possibility of lower cytotoxicity, more targeting specificity and less interaction with serum components. However, the negative charges of the anionic vector components and nucleic acids make self-assembly of the complexes challenging.

Known non-viral and viral vectors are able to deliver a plasmid DNA molecule or an RNA molecule to a target cell. In the context of a gene therapy, plasmid DNA molecules suffer from many drawbacks, for example, they contain bacterial backbone, antibiotic resistant genes and bacterial contaminants which might be toxic for a target cell or trigger an immune response.

There exists a need for an improved delivery system for gene therapy and other applications.

### DESCRIPTION

The invention provides a nanoparticle suitable for delivery of a cargo to a target cell. The invention provides a nanoparticle comprising (a) a cargo, (b) a lipid component, and (c) a targeting peptide. The nanoparticle is preferably a non-viral delivery system, such as a non-viral transfection complex. The cargo is a nucleic acid. Preferably, the cargo is a closed linear DNA molecule or a linear DNA molecule. The closed linear DNA molecule and/or the linear DNA molecule may have an enhanced resistance to nuclease (e.g. exonuclease) digestion. The closed linear DNA molecule and/or the linear DNA may comprise a cassette. The cassette may comprise a coding sequence. The coding sequence may encode a gene useful in treating a disease or disorder (e.g. a single gene disease or disorder).

The present inventors have developed a nanoparticle (e.g. a non-viral transfection complex) suitable for use in therapy (such as gene therapy). The nanoparticle of the present invention has several advantages over viral delivery systems. Firstly, the nanoparticle of the present invention is less likely to elicit an immune response, which is particularly important as repeated doses may need to be administered. In addition, the nanoparticle of the present invention can be used to deliver much larger cargos (i.e. nucleic acids) to a cell.

The nanoparticles of the present invention provide further benefits in that they are cost-effective and simple to produce on a large scale.

The nanoparticles of the present invention comprise a nucleic acid cargo (e.g. a linear DNA molecule or a closed linear DNA molecule) that has an enhanced resistance to nuclease (e.g. exonuclease) digestion, which results in a prolonged in vivo life of the cargo molecule.

Importantly, the nanoparticles of the present invention enable the efficient transfection of linear DNA molecule into a target cell or cells.

### 1. Nanoparticles and non-viral transfection complexes

The invention provides a nanoparticle suitable for delivery of a cargo to a target cell. The nanoparticle comprises: (a) a cargo; (b) a lipid component; and (c) a targeting peptide. The targeting peptide may improve targeting of a nanoparticle to a desired location. The cargo may be a nucleic acid. Preferably, the nucleic acid is linear or comprises a portion that is linear. Thus, the nanoparticle may comprise:
(a) a nucleic acid (e.g. DNA)
(b) a lipid component; and
(c) a targeting peptide comprising a targeting sequence.

The nanoparticle may comprise:
(a) a nucleic acid (e.g. DNA) comprising a linear portion;
(b) a lipid component; and
(c) a targeting peptide comprising a targeting sequence.

The nanoparticle may comprise:
(a) a linear nucleic acid (e.g. DNA);
(b) a lipid component; and
(c) a targeting peptide comprising a targeting sequence.

The nucleic acid may be a closed linear DNA molecule (e.g. a covalently-closed linear DNA molecule) or a linear DNA molecule (e.g. linear double-stranded DNA molecule).

The invention provides a nanoparticle comprising:
(a) a closed linear deoxyribonucleic acid (DNA) molecule (e.g. a covalently-closed linear DNA molecule);
(b) a lipid component; and
(c) a targeting peptide comprising a targeting sequence.

The invention also provides a nanoparticle comprising:
(a) a linear deoxyribonucleic acid (DNA) molecule;
(b) a lipid component; and
(c) a targeting peptide comprising a targeting sequence.

The linear DNA molecule may be resistant to nuclease (e.g. exonuclease) digestion. Thus, the invention provides a nanoparticle comprising:
(a) a linear deoxyribonucleic acid (DNA) molecule comprising one or more nuclease-resistance nucleotides (e.g. exonuclease-resistant nucleotides);
(b) a lipid component; and
(c) a targeting peptide comprising a targeting sequence.

The nanoparticle may be for delivery of a nucleic acid to a target cell. Preferably, the nanoparticle is for the cell-specific delivery of a nucleic acid to a target cell.

The nucleic acid may be a DNA molecule. The DNA molecule may be a linear DNA molecule or a DNA molecule comprising a linear portion. The nucleic acid may be single-stranded, double-stranded, or partially single-stranded and partially double-stranded.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid (e.g. the closed linear DNA molecule) comprises at least 500 nucleotides.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid (e.g. the closed linear DNA molecule) comprises at least 500 base pairs.

The linear DNA molecule may be a chromosome. The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides).

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The nanoparticle may be a non-viral transfection complex. Thus, the invention provides a non-viral transfection complex comprising:
(a) a closed linear deoxyribonucleic acid (DNA) molecule (e.g. a covalently-closed linear DNA molecule);
(b) a lipid component; and
(c) a targeting peptide comprising a targeting sequence.

The invention also provides a non-viral transfection complex comprising:
(a) a linear deoxyribonucleic acid (DNA) molecule comprising one or more nuclease-resistant nucleotides;
(b) a lipid component; and
(c) a targeting peptide comprising a targeting sequence.

The nanoparticle (e.g. the non-viral transfection complex) may further comprise a cationic component, an anionic component, and/or a neutral component. The nanoparticle may comprise a nucleic acid-binding component. The nucleic acid-binding component may be a nucleic acid-binding cationic component or a nucleic acid-binding neutral component. The cationic component and/or the neutral component may be used to establish a desired charge (i.e. a negative/positive ratio) of the nanoparticle. A specific charge (i.e. a Nitrogen/Phosphate ratio) may be required to facilitate or enhance cell transfection.

The nanoparticle (or a non-viral transfection complex) may comprise:
(a) a nucleic acid
(b) a lipid component;
(c) a targeting peptide comprising a targeting sequence; and
(d) a peptide comprising a nucleic acid-binding component.

The nucleic acid-binding component may be a DNA-binding component. The DNA-binding component may be a DNA-binding cationic component, or a DNA-binding neutral component. The nucleic acid-binding component may be a closed linear DNA-binding component. The closed linear DNA-binding component may be a closed linear DNA-binding cationic component, or a closed linear DNA-binding neutral component. The nucleic acid-binding component may be a linear DNA-binding component (e.g. linear double-stranded DNA-binding component). The closed linear DNA-binding component may be a closed linear DNA-binding cationic component (e.g. linear double-stranded DNA-binding cationic component), or a closed linear DNA-binding neutral component (e.g. linear double-stranded DNA-binding neutral component).

Preferably, the nucleic acid-binding component is a nucleic acid-binding cationic component (e.g. DNA-binding cationic component, closed linear DNA-binding cationic component, or linear DNA-binding cationic component).

The nucleic acid-binding cationic component may be a nucleic acid-binding polycationic component, The nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the nucleic acid-binding polycationic component comprises at least 16, at least 17 or at least 30 cationic monomers.

The nucleic acid-binding cationic component may comprise a lysine, a histidine, or an arginine. The nucleic acid- binding polycationic component may comprise a lysine, a histidine, or an arginine. The nucleic acid-binding polycationic component may comprise an oligolysine (linear or branched), an oligohistidine (linear or branched) or an oligoarginine (linear or branched). For example, the nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the nucleic acid-binding polycationic component comprises at least 16, at least 17, or at least 30 lysine residues. More preferably still, the nucleic acid-binding polycationic component comprises at least 17 lysine residues.

The nanoparticle (or a non-viral transfection complex) may comprise:
(a) a nucleic acid;
(b) a lipid component;
(c) a targeting peptide comprising a targeting sequence; and
(d) a peptide comprising a nucleic acid-binding polycationic component,
wherein the nucleic acid-binding polycationic component is oligolysine, optionally wherein the oligolysine comprises at least 17 lysine resides.

The nanoparticle (or a non-viral transfection complex) may comprise:
(a) a nucleic acid;
(b) a lipid component;
(c) a targeting peptide comprising a targeting sequence; and
(d) a peptide comprising a nucleic acid-binding polycationic component,
wherein the nucleic acid-binding polycationic component is oligolysine, optionally wherein the oligolysine comprises at least 30 lysine resides.

The nucleic acid-binding neutral component maybe be a nucleic acid-binding polyneutral component, The nucleic acid-binding polyneutral component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 neutral monomers.

The closed linear DNA-binding cationic component may be a closed linear DNA-binding polycationic component, The closed linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the closed linear DNA-binding polycationic component comprises at least 16, at least 17 or at least 30 cationic monomers.

The closed linear DNA-binding cationic component may comprise a lysine, a histidine, or an arginine. The closed linear DNA-binding polycationic component may comprise a lysine, a histidine, or an arginine. The closed linear DNA-binding polycationic component may comprise an oligolysine (linear or branched), an oligohistidine (linear or branched) or an oligoarginine (linear or branched). For example, the closed linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the closed linear DNA-binding polycationic component comprises at least 16, at least 17, or at least 30 lysine residues. More preferably still, the closed linear DNA-binding polycationic component comprises at least 17 lysine residues.

Thus, the nanoparticle (or a non-viral transfection complex) may comprise:
(a) a closed linear DNA molecule;
(b) a lipid component;
(c) a targeting peptide comprising a targeting sequence; and
(d) a peptide comprising a closed linear DNA-binding polycationic component.

The linear DNA-binding cationic component may be a linear DNA-binding polycationic component, The linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the linear DNA-binding polycationic component comprises at least 16, at least 17 or at least 30 cationic monomers.

The linear DNA-binding cationic component may comprise a lysine, a histidine, or an arginine. The linear DNA-binding polycationic component may comprise a lysine, a histidine, or an arginine. The linear DNA-binding polycationic component may comprise an oligolysine (linear or branched), an oligohistidine (linear or branched) or an oligoarginine (linear or branched). For example, the linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the linear DNA-binding polycationic component comprises at least 16, at least 17, or at least 30 lysine residues. More preferably still, the linear DNA-binding polycationic component comprises at least 17 lysine residues.

The nanoparticle (or a non-viral transfection complex) may comprise:
(a) a linear DNA molecule comprising one or more nuclease-resistant nucleotides;
(b) a lipid component;
(c) a targeting peptide comprising a targeting sequence; and
(d) a peptide comprising a closed linear DNA-binding polycationic component.

The nucleic acid-binding component may be located on the same sequence at the targeting sequence. The nucleic acid-binding component may be a part of the targeting peptide. Thus, the invention provides a nanoparticle (or a non-viral transfection complex) comprising:
(a) a nucleic acid
(b) a lipid component; and
(c) a targeting peptide comprising a targeting sequence and a nucleic acid-binding component.

Preferably, the nanoparticle (e.g. the non-viral transfection complex) comprises:
(a) a nucleic acid;
(b) a lipid component; and
(c) a targeting peptide comprising a targeting sequence and a nucleic acid-binding cationic component (e.g. a DNA-binding polycationic component).

The nanoparticle (e.g. the non-viral transfection complex) may comprise:
(a) a nucleic acid;
(b) a lipid component; and
(c) a targeting peptide comprising a targeting sequence and a nucleic acid-binding polycationic component (e.g. a DNA-binding polycationic component).

The nanoparticle (e.g. the non-viral transfection complex) may comprise:
(a) a nucleic acid;
(b) a lipid component; and
(c) a targeting peptide comprising a targeting sequence and a nucleic acid-binding polycationic component (e.g. a DNA-binding polycationic component),
wherein the nucleic acid-binding polycationic component comprises oligolysine, optionally wherein the oligolysine comprises at least 17 lysine residues.

The nanoparticle (e.g. the non-viral transfection complex) may comprise:
(a) a nucleic acid;
(b) a lipid component; and
(c) a targeting peptide comprising a targeting sequence and a nucleic acid-binding polycationic component (e.g. a DNA-binding polycationic component),
wherein the nucleic acid-binding polycationic component comprises oligolysine, optionally wherein the oligolysine comprises at least 30 lysine residues.

The nanoparticle (e.g. the non-viral transfection complex) may comprise:
(a) a closed linear DNA molecule;
(b) a lipid component; and
(c) a targeting peptide comprising a targeting sequence and a closed linear DNA-binding component (e.g. a closed linear DNA-binding polycationic component).

The nanoparticle (e.g. the non-viral transfection complex) may comprise:
(a) a closed linear DNA molecule;
(b) a lipid component; and
(c) a targeting peptide comprising a targeting sequence and a closed linear DNA-binding component (e.g. a closed linear DNA-binding polycationic component);
wherein the closed linear DNA-binding component comprises oligolysine, optionally wherein the oligolysine comprises at least 17 lysine residues.

The nanoparticle (e.g. the non-viral transfection complex) may comprise:
(a) a closed linear DNA molecule;
(b) a lipid component; and
(c) a targeting peptide comprising a targeting sequence and a closed linear DNA-binding component (e.g. a closed linear DNA-binding polycationic component);
wherein the closed linear DNA-binding component comprises oligolysine, optionally wherein the oligolysine comprises at least 30 lysine residues.

The nanoparticle (e.g. the non-viral transfection complex) may comprise:
(a) a linear DNA molecule comprising one or more nuclease-resistant nucleotides (e.g. phosphorothioated nucleotides);
(b) a lipid component; and
(c) a targeting peptide comprising a targeting sequence and a linear DNA-binding component (e.g. a linear DNA-binding polycationic component).

The nanoparticle (e.g. the non-viral transfection complex) may comprise:
(a) a linear DNA molecule comprising one or more nuclease-resistant nucleotides (e.g. phosphorothioated nucleotides);
(b) a lipid component; and
(c) a targeting peptide comprising a targeting sequence and a linear DNA-binding component (e.g. a linear DNA-binding polycationic component),
wherein the linear DNA-binding component comprises oligolysine, optionally wherein the oligolysine comprises at least 17 lysine residues.

The nanoparticle (e.g. the non-viral transfection complex) may comprise:
(a) a linear DNA molecule comprising one or more nuclease-resistant nucleotides (e.g. phosphorothioated nucleotides);
(b) a lipid component; and
(c) a targeting peptide comprising a targeting sequence and a linear DNA-binding component (e.g. a linear DNA-binding polycationic component),
wherein the linear DNA-binding component comprises oligolysine, optionally wherein the oligolysine comprises at least 30 lysine residues.

The nucleic acid-binding component may be linear or branched. The nucleic acid-binding polycationic component may be linear or branched. The nucleic acid-binding polyneutral component may be linear or branched. For example, the nucleic acid-binding polycationic component may comprise at least 16, at least 17, or at least 30 lysine residues in a linear chain. Alternatively, the nucleic acid-binding polycationic component may comprise at least 16, at least 17, or at least 30 lysine residues in a branched chain. The nucleic acid may be a nucleic acid-binding polycationic component. The nucleic acid-binding polycationic component may be linear or branched. For example, the nucleic acid-binding polycationic component (e.g. the DNA-binding polycationic component) may comprise at least 16, at least 17, or at least 30 lysine residues in a linear chain. Alternatively, the nucleic acid-binding polycationic component (e.g. the DNA-binding polycationic component) may comprise at least 16, at least 17, or at least 30 lysine residues in a branched chain.

The lipid component may be or may form a liposome. The lipid component may comprise a cationic lipid, an anionic lipid, or a neutral lipid. The lipid component may be or may form an anionic liposome, a cationic liposome, or a neutral liposome.

The liposome may comprise at least one lipid. The liposome may comprise at least one cationic lipid. The liposome may comprise at least one phospholipid. The liposome may comprise at least one cationic lipid and at least one phospholipid. The liposome may comprise at least one steroid lipid. The liposome may comprise at least one cationic lipid and at least one steroid lipid. The liposome may comprise at least one phospholipid and at least one steroid lipid. The liposome may comprise at least one cationic lipid, at least one phospholipid and at least one steroid lipid. The liposome may comprise at least one ionizable lipid. The liposome may comprise at least one anionic lipid.

The cationic lipid may be DTDTMA (ditetradecyl trimethyl ammonium), DOTMA (2,3-dioleyloxypropyl-1-trimentyl ammonium), or DHDTMA (dihexadecyl trimethyl ammonium). In addition to the cation, the cationic lipids may comprise a counter anion, for example, an inorganic counter ion, especially a pharmaceutically acceptable anion such as chloride or bromide. Preferably, the cationic lipid is DOTMA.

The lipid component may comprise a phospholipid. The term "phospholipid" refers to a lipid comprising a fatty acid chin and a phosphate group. Phospholipids are typically neutral molecules in that they do not have an overall charge, unlike a cationic lipid, which is positively charged. Phospholipids are typically zwitterionic molecules comprising both positive and negative charged components, but no overall charge. Thus, the neutral lipid may be a phospholipid. For example, the phospholipid may be DOPE (phosphatidylethanolamine or 1,2-dioleoyl-sn-glycero-3-phosphoethanolamine), or DOPC (phosphatidyl choline or 1,2-dioleoyl- sn-glycero-3-phosphoethanoltrimethylamine). Preferably, the phospholipid is DOPE.

The phospholipid may comprise a PEG moiety. The PEG moiety may have a molecular weight of from about 100 to about 10,000, optionally the PEG moiety has a molecular weight of from about 250 to about 7,500, optionally the PEG moiety has a molecular weight of from about 500 to about 5,000, optionally the PEG moiety has a molecular weight of from about 750 to about 4,000, optionally the PEG moiety has a molecular weight of from about 1,000 to about 3,000, optionally the PEG moiety has a molecular weight of approximately 2,000.

The steroid lipid may be cholesterol. The liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, at least 25%, at least 30% at least 35%, at least 40%, at least 45%, at least 50%, or at least 55% cholesterol (as defined by molar amount of cholesterol). That is to say that the liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, or at least 25% cholesterol and at least 99%, at least 98%, at least 97%, at least 96% at least 95%, at least 94%, at least 93%, at least 92%, at least 91%, at least 90%, at least 89%, at least 88%, at least 87%, at least 86%, at least 85%, at least 84%, at least 83%, at least 82%, at least 81%, at least 80% at least 79%, at least 78%, at least 77%, at least 76%, at least 75%, at least 70%, at least 65%, at least 60%, at least 55%, at least 50%, or at least 45% of other lipids in the liposome (as defined by molar ratio).

The molar ratio of at least one cationic lipid to at least one phospholipid in the nanoparticle (or non-viral transfection complex) may be 1:1, 1:2, 1:3, 1:4, 1:5, 2:1, 3:1, 4:1, or 5:1. Preferably, the molar ratio of at least one cationic lipid to at least one phospholipid in the nanoparticle (or non-viral transfection complex) is 1:1 or 2:1. For example, the molar ratio of DOTMA to DOPE in the nanoparticle (or non-viral transfection complex) may be 1:1. That is to say that the molar amount of DOTMA and DOPE in the nanoparticle is the same. The molar ratio of DOTMA to DOPE in the nanoparticle (or non-viral transfection complex) may be 2:1. That is to say that the molar amount of DOTMA is twice the molar amount of DOPE.

The targeting sequence may comprise at least 3, at least 4, at least 5, at least 6, at least 7, at least 8, at least 9, at least 10, at least 11, at least 12, at least 13 or at least 14 amino acids. The targeting sequence may comprise 3-30, 4-20, 5-17, 6-15 or 7-14 amino acids. Preferably, the targeting sequence comprises 7-14 amino acids. For example, the targeting sequence may comprise 7 or 12 amino acids.

The targeting sequence may target a brain cell, a kidney cell, a muscle cell, a skin cell (e.g. a skin epithelial cell) or a spleen cell. The targeting sequence may be a brain cell targeting sequence. The targeting sequence may target a cell-surface receptor or a biologically active molecule present on the surface of brain cells. The targeting sequence may be a kidney cell targeting sequence. The targeting sequence may target a cell-surface receptor or a biologically active molecule present on the surface of kidney cells. The targeting sequence may be a muscle cell targeting sequence. The targeting sequence may target a cell-surface receptor or a biologically active molecule present on the surface of muscle cells. The targeting sequence may be a skin cell (e.g. a skin epithelial cell) targeting sequence. The targeting sequence may target a cell-surface receptor or a biologically active molecule present on the surface of skin cells (e.g. skin epithelial cells). The targeting sequence may be a spleen cell targeting sequence. The targeting sequence may target a cell-surface receptor or a biologically active molecule present on the surface of spleen cells.

The term "brain cell targeting sequence" refers to a sequence that has the ability to target, bind and/or interact with a brain cell. For example, the brain cell targeting sequence may target, bind and/or interact with a receptor on a muscle cell, or a biologically active molecule present on the surface of brain cells. The term "kidney cell targeting sequence" refers to a sequence that has the ability to target, bind and/or interact with a kidney cell. For example, the kidney cell targeting sequence may target, bind and/or interact with a receptor on a kidney cell, or a biologically active molecule present on the surface of kidney cells. The term "muscle cell targeting sequence" refers to a sequence that has the ability to target, bind and/or interact with a muscle cell. For example, the muscle cell targeting sequence may target, bind and/or interact with a receptor on a muscle cell, or a biologically active molecule present on the surface of muscle cells. The term "skin cell targeting sequence" refers to a sequence that has the ability to target, bind and/or interact with a skin cell. For example, the skin cell targeting sequence may target, bind and/or interact with a receptor on a skin cell, or a biologically active molecule present on the surface of skin cells. The term "spleen cell targeting sequence" refers to a sequence that has the ability to target, bind and/or interact with a spleen cell. For example, the spleen cell targeting sequence may target, bind and/or interact with a receptor on a spleen cell, or a biologically active molecule present on the surface of spleen cells.

The targeting sequence may be a cell type-specific targeting sequence (e.g. a brain-specific targeting sequence, a kidney-specific targeting sequence, a muscle-specific targeting sequence, a skin-specific targeting sequence or a spleen-specific targeting sequence). The term "specific" refers to the ability to preferentially target, bind and/or interact with a given target, for example, a receptor on a brain, kidney, muscle, skin or spleen cell, over other targets. Preferential targeting, binding and/or interacting with may be assessed by measuring the binding affinity of a sequence for a target receptor on a target cell in comparison to a target on a different cell. Similarly, preferential targeting, binding and/or interacting with may be measured by calculating a dissociation rate (of a sequence from a target molecule). Preferential targeting may be assessed by comparing the amount of binding events for each one of the target cells. For example, the brain-specific targeting sequence may bind to a brain cell more often than any other cell type. The brain-specific targeting sequence may bind with higher affinity for a brain cell (or a receptor on a brain cell) than any other cell (or receptor) type. For example, the kidney-specific targeting sequence may bind to a kidney cell more often than any other cell type. The kidney-specific targeting sequence may bind with higher affinity for a kidney cell (or a receptor on a kidney cell) than any other cell (or receptor) type. For example, the muscle-specific targeting sequence may bind to a muscle cell more often than any other cell type. The muscle-specific targeting sequence may bind with higher affinity for a muscle cell (or a receptor on a muscle cell) than any other cell (or receptor) type. For example, the skin-specific targeting sequence may bind to a skin cell more often than any other cell type. The skin-specific targeting sequence may bind with higher affinity for a skin cell (or a receptor on a skin cell) than any other cell (or receptor) type. For example, the spleen-specific targeting sequence may bind to a spleen cell more often than any other cell type. The spleen-specific targeting sequence may bind with higher affinity for a spleen cell (or a receptor on a spleen cell) than any other cell (or receptor) type.

The nanoparticle may comprise:
(a) a closed linear DNA molecule (e.g. a covalently-closed linear DNA molecule);
(b) a lipid component; and
(c) a targeting peptide comprising a brain cell targeting sequence.

The nanoparticle may comprise:
(a) a linear DNA molecule comprising one or more nuclease-resistance nucleotides;
(b) a lipid component; and
(c) a targeting peptide comprising a brain cell targeting sequence.

The nanoparticle may comprise:
(a) a closed linear DNA molecule (e.g. a covalently-closed linear DNA molecule);
(b) a lipid component; and
(c) a targeting peptide comprising a kidney cell targeting sequence.

The nanoparticle may comprise:
(a) a linear DNA molecule comprising one or more nuclease-resistance nucleotides;
(b) a lipid component; and
(c) a targeting peptide comprising a kidney cell targeting sequence.

The nanoparticle may comprise:
(a) a closed linear DNA molecule (e.g. a covalently-closed linear DNA molecule);
(b) a lipid component; and
(c) a targeting peptide comprising a muscle cell targeting sequence.

The nanoparticle may comprise:
(a) a linear DNA molecule comprising one or more nuclease-resistance nucleotides;
(b) a lipid component; and
(c) a targeting peptide comprising a muscle cell targeting sequence.

The nanoparticle may comprise:
(a) a closed linear DNA molecule (e.g. a covalently-closed linear DNA molecule);
(b) a lipid component; and
(c) a targeting peptide comprising a skin cell targeting sequence.

The nanoparticle may comprise:
(a) a linear DNA molecule comprising one or more nuclease-resistance nucleotides;
(b) a lipid component; and
(c) a targeting peptide comprising a skin cell targeting sequence.

The nanoparticle may comprise:
(a) a closed linear DNA molecule (e.g. a covalently-closed linear DNA molecule);
(b) a lipid component; and
(c) a targeting peptide comprising a spleen cell targeting sequence.

The nanoparticle may comprise:
(a) a linear DNA molecule comprising one or more nuclease-resistance nucleotides;
(b) a lipid component; and
(c) a targeting peptide comprising a spleen cell targeting sequence.

The muscle cell targeting sequence may be a skeletal muscle cell targeting sequence, a cardiac muscle cell targeting sequence, or a smooth muscle cell targeting sequence. Preferably, the muscle cell targeting sequence is a skeletal muscle cell targeting sequence.

The muscle cell targeting sequence may preferentially bind to the skeletal muscle cell over the smooth muscle cell or the cardiac muscle cell. That is to say that the muscle cell targeting sequence is a skeletal muscle cell-specific targeting sequence. The muscle cell targeting sequence may preferentially bind to the cardiac muscle cell over a smooth muscle cell. That is to say that the muscle cell targeting sequence is a cardiac muscle cell-specific targeting sequence.

The targeting sequence may comprise at least 3, at least 4, at least 5 or at least 6 contiguous amino acids of SEQ ID NO: 1 or a variant thereof comprising one or more conservative amino acid substitutions. The targeting sequence may be SEQ ID NO: 1 or a variant thereof comprising one or more conservative amino acid substitutions. The targeting sequence may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 1. The targeting sequence may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 1.

The targeting sequence (e.g. the muscle cell targeting sequence) may comprise at least 3, at least 4, at least 5 or at least 6 contiguous amino acids of SEQ ID NO: 6 or a variant thereof comprising one or more conservative amino acid substitutions. The targeting sequence (e.g. the muscle cell targeting sequence) may be SEQ ID NO: 6 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 6. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 6.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 7 or a variant thereof comprising one or more conservative amino acid substitutions. The targeting sequence (e.g. the muscle cell targeting sequence) may be SEQ ID NO: 7 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 7. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 7.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 8 or a variant thereof comprising one or more conservative amino acid substitutions. The targeting sequence (e.g. the muscle cell targeting sequence) may be SEQ ID NO: 8 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 8. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 8.

The targeting sequence may be any sequence listed in Table 1 or a variant thereof comprising one or more conservative amino acid substitutions.

**Table 1. Exemplary target sequences.**

| SEQ ID NO | Sequence | Peptide name |
|---|---|---|
| 1 | YGLPHKF | |
| 6 | ASSLNIA | MD1 |
| 7 | RRQPPRSISSHP | MD2 |
| 8 | SKTFNTHPQSTP | MD3 |

Thus, the invention provides a nanoparticle (or a non-viral transfection complex) comprising:
(a) a nucleic acid;
(b) a lipid component; and
(c) a targeting peptide comprising a targeting sequence and a nucleic acid-binding component,
wherein the targeting sequence is SEQ ID NO: 1 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding component, wherein the targeting sequence is SEQ ID NO: 1 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding component,
wherein the targeting sequence is SEQ ID NO: 1 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding polycationic component, wherein the targeting sequence is SEQ ID NO: 1 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding polycationic component, wherein the targeting sequence is SEQ ID NO: 1 or a variant thereof comprising one or more conservative amino acid substitutions.

The invention also provides a nanoparticle (or a non-viral transfection complex) comprising:
(a) a nucleic acid;
(b) a lipid component; and
(c) a targeting peptide comprising a targeting sequence and a nucleic acid-binding component,
wherein the targeting sequence is SEQ ID NO: 6 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding component, wherein the targeting sequence is SEQ ID NO: 6 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding component,
wherein the targeting sequence is SEQ ID NO: 6 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding polycationic component, wherein the targeting sequence is SEQ ID NO: 6 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding polycationic component, wherein the targeting sequence is SEQ ID NO: 6 or a variant thereof comprising one or more conservative amino acid substitutions.

The invention also provides a nanoparticle (or a non-viral transfection complex) comprising:
(a) a nucleic acid;
(b) a lipid component; and
(c) a targeting peptide comprising a targeting sequence and a nucleic acid-binding component,
wherein the targeting sequence is SEQ ID NO: 7 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding component, wherein the targeting sequence is SEQ ID NO: 7 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding component,
wherein the targeting sequence is SEQ ID NO: 7 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding polycationic component, wherein the targeting sequence is SEQ ID NO: 7 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding polycationic component, wherein the targeting sequence is SEQ ID NO: 7 or a variant thereof comprising one or more conservative amino acid substitutions.

The invention also provides a nanoparticle (or a non-viral transfection complex) comprising:
(a) a nucleic acid;
(b) a lipid component; and
(c) a targeting peptide comprising a targeting sequence and a nucleic acid-binding component,
wherein the targeting sequence is SEQ ID NO: 8 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding component, wherein the targeting sequence is SEQ ID NO: 8 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding component,
wherein the targeting sequence is SEQ ID NO: 8 or a variant thereof comprising one or more conservative amino acid substitutions.

The nanoparticle (or a non-viral transfection complex) may comprise:
(a) a closed linear DNA molecule (e.g. a covalently-closed linear DNA molecule);
(b) a lipid component; and
(c) a targeting peptide comprising a targeting sequence and a linear closed DNA-binding polycationic component, wherein the targeting sequence is SEQ ID NO: 8 or a variant thereof comprising one or more conservative amino acid substitutions.

The nanoparticle (or a non-viral transfection complex) may comprise:
(a) a linear DNA molecule comprising one or more nuclease-resistance nucleotides (e.g. exonuclease-resistant nucleotides);
(b) a lipid component; and
(c) a targeting peptide comprising a targeting sequence and a linear DNA-binding polycationic component, wherein the targeting sequence is SEQ ID NO: 8 or a variant thereof comprising one or more conservative amino acid substitutions.

The targeting peptide may comprise at least 4, at least 5, at least 6, at least 7, at least 8, at least 9, at least 10, at least 11, at least 12, at least 13, or at least 14 amino acids. The targeting sequence may comprise 4-100, 5-70, 6-50, or 7-40 amino acids.

The targeting peptide may comprise at least 4, at least 5, at least 6, at least 7, or at least 8 contiguous amino acids of SEQ ID NO: 2 or a variant thereof comprising one or more conservative amino acid substitutions. The targeting peptide may be SEQ ID NO: 2 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 2. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 2.

The targeting peptide may comprise at least 6, at least 7, at least 8, at least 9, or at least 10 contiguous amino acids of SEQ ID NO: 3 or a variant thereof comprising one or more conservative amino acid substitutions. The targeting peptide may be SEQ ID NO: 3 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 3. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 3.

The targeting peptide may comprise at least 18, at least 19, at least 20, at least 21, at least 22, at least 23, at least 24, at least 25, or at least 26 contiguous amino acids of SEQ ID NO: 4 or a variant thereof comprising one or more conservative amino acid substitutions. The targeting peptide may be SEQ ID NO: 4 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:4. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:4.

The targeting peptide may comprise at least 32, at least 33, at least 34, at least 35, at least 36, at least 37, least 38, at least 39, or at least 40 contiguous amino acids of SEQ ID NO: 5 or a variant thereof comprising one or more conservative amino acid substitutions. The targeting peptide may be SEQ ID NO: 5 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:5. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:5.

The targeting peptide may comprise at least 4, at least 5, at least 6, at least 7, or at least 8 contiguous amino acids of SEQ ID NO: 9 or a variant thereof comprising one or more conservative amino acid substitutions. The targeting peptide may be SEQ ID NO: 9 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 9. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 9.

The targeting peptide may comprise at least 6, at least 7, or at least 8, at least 9, at least 10 contiguous amino acids of SEQ ID NO: 10 or a variant thereof comprising one or more conservative amino acid substitutions. The targeting peptide may be SEQ ID NO: 10 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 10. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 10.

The targeting peptide may comprise at least 8, at least 9, at least 10, at least 11, at least 12, at least 13, or at least 14 contiguous amino acids of SEQ ID NO: 11 or a variant thereof comprising one or more conservative amino acid substitutions. The targeting peptide may be SEQ ID NO: 11 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 11. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 11

The targeting peptide may comprise at least 20, at least 21, at least 22, at least 23, least 24, at least 25, at least 26, at least 27, at least 28, at least 29, or at least 30 contiguous amino acids of SEQ ID NO: 12 or a variant thereof comprising one or more conservative amino acid substitutions. The targeting peptide may be SEQ ID NO: 12 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 12. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 12.

The targeting peptide may comprise at least 34, at least 35, at least 36, at least 37, least 38, at least 39, at least 40, at least 41, at least 42, at least 43, or at least 44 contiguous amino acids of SEQ ID NO: 13 or a variant thereof comprising one or more conservative amino acid substitutions. The targeting peptide may be SEQ ID NO: 13 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 13. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 13.

The targeting peptide may comprise at least 18, at least 19, at least 20, at least 21, at least 22, at least 23, at least 24, at least 25, or at least 26 contiguous amino acids of SEQ ID NO: 14 or a variant thereof comprising one or more conservative amino acid substitutions. The targeting peptide may be SEQ ID NO: 14 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 14. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 14.

The targeting peptide may comprise at least 32, at least 33, at least 34, at least 35, at least 36, at least 37, at least 38, at least 39, or at least 40 contiguous amino acids of SEQ ID NO: 15 or a variant thereof comprising one or more conservative amino acid substitutions. The targeting peptide may be SEQ ID NO: 15 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 15. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 15.

The targeting peptide may comprise at least 4, at least 5, at least 6, at least 7, or at least 8 contiguous amino acids of SEQ ID NO: 16 or a variant thereof comprising one or more conservative amino acid substitutions. The targeting peptide may be SEQ ID NO: 16 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 16. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 16.

The targeting peptide may comprise at least 16, at least 17, at least 18, at least 19, at least 20, at least 21, at least 22, at least 23, or at least 24 contiguous amino acids of SEQ ID NO: 17 or a variant thereof comprising one or more conservative amino acid substitutions. The targeting peptide may be SEQ ID NO: 17 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 17. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 17.

The targeting peptide may comprise at least 30, at least 31, at least 32, at least 33, at least 34, at least 35, at least 36, at least 37, or at least 38 contiguous amino acids of SEQ ID NO: 18 or a variant thereof comprising one or more conservative amino acid substitutions. The targeting peptide may be SEQ ID NO: 18 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 18. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 18.

The targeting peptide may comprise at least 7, at least 8, at least 9, at least 10, at least 11, at least 12, or at least 13, contiguous amino acids of SEQ ID NO: 19 or a variant thereof comprising one or more conservative amino acid substitutions. The targeting peptide may be SEQ ID NO: 19 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 19. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 19.

The targeting peptide may comprise at least 9, at least 10, at least 11, at least 12, at least 13, at least 14, or at least 15 contiguous amino acids of SEQ ID NO: 20 or a variant thereof comprising one or more conservative amino acid substitutions. The targeting peptide may be SEQ ID NO: 20 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 20. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 20.

The targeting peptide may comprise at least 11, at least 12, at least 13, at least 14, at least 15, at least 16, at least 17, at least 18, or at least 19 contiguous amino acids of SEQ ID NO: 21 or a variant thereof comprising one or more conservative amino acid substitutions. The targeting peptide may be SEQ ID NO: 21 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 21. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 21.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 22 or a variant thereof comprising one or more conservative amino acid substitutions. The targeting peptide may be SEQ ID NO: 22 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 22. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 22.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 23 or a variant thereof comprising one or more conservative amino acid substitutions. The targeting peptide may be SEQ ID NO: 23 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 23. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 23.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 24 or a variant thereof comprising one or more conservative amino acid substitutions. The targeting peptide may be SEQ ID NO: 24 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 24. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 24.

The targeting peptide may comprise at least 34, least 35, at least 36, at least 37, least 38, at least 39, at least 40, at least 41, at least 42, at least 43, at least 44, or at least 45 contiguous amino acids of SEQ ID NO: 25 or a variant thereof comprising one or more conservative amino acid substitutions. The targeting peptide may be SEQ ID NO: 25 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 25. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 25.

The targeting peptide may comprise at least 6, at least 7, at least 8, at least 9, or at least 10, at least 11, at least 12, or at least 13 contiguous amino acids of SEQ ID NO: 26 or a variant thereof comprising one or more conservative amino acid substitutions. The targeting peptide may be SEQ ID NO: 26 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 26. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 26.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 27 or a variant thereof comprising one or more conservative amino acid substitutions. The targeting peptide may be SEQ ID NO: 27 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 27. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 27.

The targeting peptide may comprise at least 33, least 34, at least 35, least 36, at least 37, at least 38, least 39, at least 40, at least 41, at least 42, at least 43, or at least 44 contiguous amino acids of SEQ ID NO: 28 or a variant thereof comprising one or more conservative amino acid substitutions. The targeting peptide may be SEQ ID NO: 28 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 28. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 28.

The targeting peptide may comprise at least 6, at least 7, at least 8, at least 9, or at least 10, at least 11, at least 12, or at least 13 contiguous amino acids of SEQ ID NO: 29 or a variant thereof comprising one or more conservative amino acid substitutions. The targeting peptide may be SEQ ID NO: 29 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 29. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 29.

The targeting peptide may comprise at least 8, at least 9, or at least 10, at least 11, at least 12, at least 13, at least 14, or at least 15 contiguous amino acids of SEQ ID NO: 30 or a variant thereof comprising one or more conservative amino acid substitutions. The targeting peptide may be SEQ ID NO: 30 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 30. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 30.

The targeting peptide may comprise at least 11, at least 12, at least 13, at least 14, at least 15, at least 16, at least 17, at least 18, or at least 19 contiguous amino acids of SEQ ID NO: 31 or a variant thereof comprising one or more conservative amino acid substitutions. The targeting peptide may be SEQ ID NO: 31 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 31. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 31.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 32 or a variant thereof comprising one or more conservative amino acid substitutions. The targeting peptide may be SEQ ID NO: 32 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 32. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 32.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 33 or a variant thereof comprising one or more conservative amino acid substitutions. The targeting peptide may be SEQ ID NO: 33 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 33. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 33.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 34 or a variant thereof comprising one or more conservative amino acid substitutions. The targeting peptide may be SEQ ID NO: 34 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 34. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 34.

The targeting peptide may comprise at least 35, at least 36, at least 37, least 38, at least 39, at least 40, at least 41, at least 42, at least 43, at least 44, or at least 45 contiguous amino acids of SEQ ID NO: 35 or a variant thereof comprising one or more conservative amino acid substitutions. The targeting peptide may be SEQ ID NO: 35 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 35. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 35.

The targeting peptide may comprise at least 6, at least 7, at least 8, at least 9, or at least 10, at least 11, at least 12, or at least 13 contiguous amino acids of SEQ ID NO: 36 or a variant thereof comprising one or more conservative amino acid substitutions. The targeting peptide may be SEQ ID NO: 36 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 36. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 36.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 37 or a variant thereof comprising one or more conservative amino acid substitutions. The targeting peptide may be SEQ ID NO: 37 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 37. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 37.

The targeting peptide may comprise at least 32, least 33, at least 34, least 35, at least 36, at least 37, least 38, at least 39, at least 40, at least 41, at least 42, or at least 43 contiguous amino acids of SEQ ID NO: 38 or a variant thereof comprising one or more conservative amino acid substitutions. The targeting peptide may be SEQ ID NO: 38 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 38. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 38.

By "sequence identity" or "sequence similarity" is meant that the identity or similarity, respectively, between two or more amino acid sequences, or two or more nucleotide sequences, is expressed in terms of the identity or similarity between the sequences. Sequence identity can be measured in terms of "percentage (%) identity," in which a higher percentage indicates greater identity shared between the sequences. Sequence similarity can be measured in terms of percentage similarity (which takes into account conservative amino acid substitutions); the higher the percentage, the more similarity shared between the sequences.

The peptides described herein may comprise conservative amino acid substitutions at one or more amino acid residues, e.g. at essential or non-essential amino acid residues. A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagines, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The targeting peptide may comprise any one of the sequences of Table 2.

**Table 2. Exemplary targeting peptides.**

| SEQ ID NO | Sequence | Peptide name |
|---|---|---|
| 2 | CYGLPHKFC | |
| 3 | GACYGLPHKFC | |
| 4 | KKKKKKKKKKKKKKKKGACYGLPHKFC | Peptide Y |
| 5 | KKKKKKKKKKKKKKKKKKKKKKKKKKKKKKGACYGLPHKFC | Peptide Y30 |
| 9 | CASSLNIAC | |
| 10 | GACASSLNIAC | |
| 11 | RVRRGACASSLNIAC | |
| 12 | KKKKKKKKKKKKKKKKRVRRGACASSLNIAC | MD1CC |
| 13 | KKKKKKKKKKKKKKKKKKKKKKKKKKKKKKRVRRGACASSLNIAC | |
| 14 | KKKKKKKKKKKKKKKKGACASSLNIAC | MD1C |
| 15 | KKKKKKKKKKKKKKKKKKKKKKKKKKKKKKGACASSLNIAC | |
| 16 | GAASSLNIA | |
| 17 | KKKKKKKKKKKKKKKKGAASSLNIA | MD1L |
| 18 | KKKKKKKKKKKKKKKKKKKKKKKKKKKKKKGAASSLNIA | |
| 19 | CRRQPPRSISSHPC | |
| 20 | GACRRQPPRSISSHPC | |
| 21 | RVRRGACRRQPPRSISSHPC | |
| 22 | KKKKKKKKKKKKKKKKRVRRGACRRQPPRSISSHPC | MD2CC |
| 23 | KKKKKKKKKKKKKKKKKKKKKKKKKKKKKKRVRRGACRRQPPRSISSHP C | |
| 24 | KKKKKKKKKKKKKKKKGACRRQPPRSISSHPC | MD2C |
| 25 | KKKKKKKKKKKKKKKKKKKKKKKKKKKKKKGACRRQPPRSISSHPC | |
| 26 | GARRQPPRSISSHP | |
| 27 | KKKKKKKKKKKKKKKKGARRQPPRSISSHP | MD2L |
| 28 | KKKKKKKKKKKKKKKKKKKKKKKKKKKKKKGARRQPPRSISSHP | |
| 29 | CSKTFNTHPQSTPC | |
| 30 | GACSKTFNTHPQSTPC | |
| 31 | RVRRGACSKTFNTHPQSTPC | |
| 32 | KKKKKKKKKKKKKKKKRVRRGACSKTFNTHPQSTPC | MD3CC |
| 33 | KKKKKKKKKKKKKKKKKKKKKKKKKKKKKKRVRRGACSKTFNTHPQSTP | |
| | C | |
| 34 | KKKKKKKKKKKKKKKKGACSKTFNTHPQSTPC | MD3C |
| 35 | KKKKKKKKKKKKKKKKKKKKKKKKKKKKKKGACSKTFNTHPQSTPC | |
| 36 | GASKTFNTHPQSTP | |
| 37 | KKKKKKKKKKKKKKKKGASKTFNTHPQSTP | MD3L |
| 38 | KKKKKKKKKKKKKKKKKKKKKKKKKKKKKKGASKTFNTHPQSTP | |

The targeting peptide may comprise a cyclic region, a branched region, and/or a linear region.

The targeting peptide comprising the cyclic region may be formed by the provision of at least two cysteine residues in the peptide, thus enabling the formation of a disulphide bond. Thus, the targeting peptide may comprise two or more cysteine residues that are capable of forming one or more disulphide bond(s). Preferably, the two or more cysteine residues flank the targeting sequence. For example, if the targeting sequence is ASSLNIA (SEQ ID NO: 6), the targeting peptide may comprise a sequence: CASSLNIAC (SEQ ID NO: 9).

The targeting peptide may comprise a linker. The linker may be cleavable or non-cleavable.

The linker may comprise at least 1, at least 2, at least 3, at least 4, at least 5, at least 6, at least 7, at least 8, at least 9, at least 10, at least 11 or at least 12 amino acids. The linker may comprise 2-10 amino acids or 4-8 amino acids. The amino acids may be naturally occurring or non-naturally occurring. They may have L- or D- configuration. The amino acids may be the same or different. The use of multiple lysine residues (or other cationic amino acids suitable for use in the nucleic acid-binding polycationic component) should generally be avoided in the linker as oligo-lysine sequences have activity as a nucleic acid-binding polycationic component.

The linker may comprise a cleavable portion that is susceptible to cleavage within a cell. The linker that comprises a cleavable portion that is susceptible to cleavage within a cell may be susceptible to cleavage within the endosome, lysosome, and/or cytoplasm of a cell. The expression "susceptible to cleavage" refers to a linker that is susceptible to cleavage over a timescale during which the remaining elements of the targeting peptide are intact. Thus, the linker may be cleaved more rapidly than the cellular peptide-degradation pathways take effect. The cleavable portion may comprise from 3 to 6 amino acids, for example 4 amino acids. The linker may include the amino acid sequence RVRR (SEQ ID NO: 39) as a cleavable portion. The amino acid sequence RVRR is susceptible to enzymatic cleavage by the endosomal protease furin. The cleavable portion of the linker may be attached to a nucleic acid-binding component. The cleavable portion of the linker may be cleavable by a protease. The protease may be cathepsin (e.g. serine, cysteine, aspartic-type), furin, a lysosomal protease or an endosomal protease.

The targeting peptide may comprise a spacer. The spacer may be either a peptide, that is to say, it comprises amino acid residues, or a polyethyleneglycol group, or a mixture of the two. The amino acids may be naturally occurring or non-naturally occurring. They may have L- or D-configuration. The spacer may have one or more amino acids. The spacer may comprise at least 1, at least 2, at least 3, at least 4, at least 5, at least 6, at least 7, at least 8, at least 9, at least 10, at least 11 or at least 12 amino acids.

The spacer may comprise 1-7 amino acids, preferably 2-5 amino acids. The amino acids may be the same or different. The spacer may comprise the dipeptide glycine-glycine (GG), glycine-alanine (GA) or alanine-alanine (AA). The spacer may comprise a hydrophobic spacer. The spacer may include the amino acid sequence XSX in which X is c-aminocaproic acid (c-Ahx) also known as 6-aminohexanoic acid, a synthetic, i.e. non-naturally occurring, amino acid. Aminocaproic acid functions as a hydrophobic spacer. The spacer may comprise GG, GA, AA, XSXGG (SEQ ID NO: 40), XSXGA (SEQ ID NO: 41) or XSXAA (SEQ ID NO: 43). Preferably, the spacer comprises GA or GG. The spacer may be located at the end of the linker in a targeting peptide. The spacer may be attached to a targeting sequence or a targeting sequence flanked by cysteine residues. Preferably, the spacer links the linker and the targeting sequence (which is optionally flanked by the cysteine residues).

The targeting peptide may have a structure: A-B-C-D, wherein component A is a nucleic acid-binding component, component B is a linker, component C is a spacer and component D is a targeting sequence (optionally flanked by the cysteine residues).

The targeting peptide may have a structure: A-B-D, wherein component A is a nucleic acid-binding component, component B is a linker and component D is a targeting sequence (optionally flanked by the cysteine residues).

The targeting peptide may have a structure: A-D, wherein component A is a nucleic acid-binding component and component D is a targeting sequence (optionally flanked by the cysteine residues).

The nanoparticle (e.g. the non-viral transfection complex) may have a particle size of less than 500 nm, for example less than 250 nm or less than 100 nm. In a population of particles there will be some variation in particle size but the above criteria will be taken as met if at least 70%, at least 80% or at least 90% of the particles are of less than 500 nm, for example less than 250nm or less than 100 nm. Preferably, in a population of particles, at least 80% of the particles are less than 500 nm, for example less than 250nm or less than 100 nm.

The nanoparticle (e.g. the non-viral transfection complex) may have a charge ratio (i.e. Nitrogen/Phosphate (N/P) molar ratio) of 2.0-13.0, 3.0-12.0, 4.0-11.0 or 4.0-12.0. The nanoparticle (e.g. the non-viral transfection complex) may have a charge ratio (i.e. N/P ratio) of about 4.2, about 4.5, about 4.7, about 5.2, about 5.5, about 5.7 about 6.5, about 7.5, about 8.5, about 9.5 or about 10.5. Preferably, the nanoparticle has a charge ratio (i.e. N/P ratio) of 7.0-11.0 or 3.0-8.0.

For example, if the nucleic acid-binding component of the nanoparticle is a nucleic acid-binding polycationic component comprising 16 lysine residues, the nanoparticle may have a charge ratio (i.e. N/P ratio) of 3.0-12.0 or 4.0-12.0, preferably 7.0-11.0. For example, if the nucleic acid-binding component of the nanoparticle is a nucleic acid-binding polycationic component comprising 16 lysine residues, the nanoparticle may have a charge ratio (i.e. N/P ratio) of about 7.5, about 8.5, or about 10.5.

For example, if the nucleic acid-binding component is a nucleic acid-binding polycationic component comprising 30 lysine residues, the nanoparticle may have a charge ratio (i.e. N/P ratio) of 3.0-8.0 or 4.0-8.0. For example, if the nucleic acid-binding component is a nucleic acid-binding polycationic component comprising 30 lysine residues, the nanoparticle may have a charge ratio (i.e. N/P ratio) of about 4.2, about 4.5, about 4.7, about 5.2, about 5.5, about 5.7 about 6.5, or about 7.5 .

The nanoparticle (e.g. the non-viral transfection complex) may have a charge ratio (i.e. Nitrogen/Phosphate (N/P) molar ratio) of 7.0-13.0, 7.2-13.0, 7.5-13.0, or 8.0-13.0.

The charge ratio (N/P ratio) is calculated from the molar amount of each free amine group(s) (N) in the components of the nanoparticle to the phosphate groups (P) in the components of the nanoparticle. For example, the free amine group(s) may come from the targeting peptide and the lipid component and the phosphate group(s) may come from the phosphate groups in the nucleic acid molecule (e.g. DNA molecule) (P). The charge ratio is typically driven by the mass of the targeting peptide.

The term "about" as used herein for numerical parameters refers to a value within 10% of the underlying parameter (i.e. plus or minus 10%). For example, a charge ratio of "about 4.5" can include charge ratios between 4.1 - 5.0, including charge ratios 4.1 and 5.0.

The nanoparticle (e.g. the non-viral delivery complex) may deliver the nucleic acid cargo to a target cell with a transfection efficiency of at least 5%, at least 10%, at least 15%, at least 20%, at least 25%, at least 30%, at least 35%, at least 40%, at least 50%, at least 52%, at least 55%, at least 60%, at least 65%, at least 70%, at least 75%, at least 80%, at least 85%, at least 90%, at least 95%, or at least 98%. Preferably, the transfection efficiency is at least 15%.

### 2. Targeting peptides

The invention provides a targeting peptide comprising a targeting sequence. Targeting peptide may be suitable for use in the nanoparticle (e.g. the non-viral transfection complex) described herein. The targeting peptide may comprise a nucleic acid-binding component.

The invention provides a targeting peptide comprising:
(a) a targeting sequence; and
(b) a nucleic acid-binding component.

The targeting sequence may comprise at least 3, at least 4, at least 5 or at least 6 contiguous amino acids of SEQ ID NO: 1 or a variant thereof comprising one or more conservative amino acid substitutions. The targeting sequence may be SEQ ID NO: 1 or a variant thereof comprising one or more conservative amino acid substitutions. The targeting sequence may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 1. The targeting sequence may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 1.

The targeting sequence (e.g. the muscle cell targeting sequence) may comprise at least 3, at least 4, at least 5 or at least 6 contiguous amino acids of SEQ ID NO: 6 or a variant thereof comprising one or more conservative amino acid substitutions. The targeting sequence (e.g. the muscle cell targeting sequence) may be SEQ ID NO: 6 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 6. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 6.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 7 or a variant thereof comprising one or more conservative amino acid substitutions. The targeting sequence (e.g. the muscle cell targeting sequence) may be SEQ ID NO: 7 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 7. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 7.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 8 or a variant thereof comprising one or more conservative amino acid substitutions. The targeting sequence (e.g. the muscle cell targeting sequence) may be SEQ ID NO: 8 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 8. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 8.

Thus, the invention provides a targeting peptide comprising a targeting sequence and nucleic acid-binding component, wherein the targeting sequence is SEQ ID NO: 1 or a variant thereof comprising one or more conservative amino acid substitutions.

Thus, the invention provides a targeting peptide comprising a targeting sequence and nucleic acid-binding component, wherein the targeting sequence is SEQ ID NO: 6 or a variant thereof comprising one or more conservative amino acid substitutions.

Thus, the invention provides a targeting peptide comprising a targeting sequence and nucleic acid-binding component, wherein the targeting sequence is SEQ ID NO: 7 or a variant thereof comprising one or more conservative amino acid substitutions.

Thus, the invention provides a targeting peptide comprising a targeting sequence and nucleic acid-binding component, wherein the targeting sequence is SEQ ID NO: 8 or a variant thereof comprising one or more conservative amino acid substitutions.

Thus, the invention provides a targeting peptide comprising a targeting sequence and nucleic acid-binding cationic component, wherein the targeting sequence is SEQ ID NO: 1 or a variant thereof comprising one or more conservative amino acid substitutions.

Thus, the invention provides a targeting peptide comprising a targeting sequence and nucleic acid-binding cationic component, wherein the targeting sequence is SEQ ID NO: 6 or a variant thereof comprising one or more conservative amino acid substitutions.

Thus, the invention provides a targeting peptide comprising a targeting sequence and nucleic acid-binding cationic component, wherein the targeting sequence is SEQ ID NO: 7 or a variant thereof comprising one or more conservative amino acid substitutions.

Thus, the invention provides a targeting peptide comprising a targeting sequence and nucleic acid-binding cationic component, wherein the targeting sequence is SEQ ID NO: 8 or a variant thereof comprising one or more conservative amino acid substitutions.

Thus, the invention provides a targeting peptide comprising a targeting sequence and nucleic acid-binding polycationic component, wherein the targeting sequence is SEQ ID NO: 1 or a variant thereof comprising one or more conservative amino acid substitutions.

Thus, the invention provides a targeting peptide comprising a targeting sequence and nucleic acid-binding polycationic component, wherein the targeting sequence is SEQ ID NO: 6 or a variant thereof comprising one or more conservative amino acid substitutions.

Thus, the invention provides a targeting peptide comprising a targeting sequence and nucleic acid-binding polycationic component, wherein the targeting sequence is SEQ ID NO: 7 or a variant thereof comprising one or more conservative amino acid substitutions.

Thus, the invention provides a targeting peptide comprising a targeting sequence and nucleic acid-binding polycationic component, wherein the targeting sequence is SEQ ID NO: 8 or a variant thereof comprising one or more conservative amino acid substitutions.

The targeting peptide may comprise a targeting sequence and a closed linear DNA-binding component (e.g. a closed linear DNA-binding polycationic component), wherein the targeting sequence is SEQ ID NO: 1, SEQ ID NO: 6, SEQ ID NO: 7, or SEQ ID NO: 8, or a variant thereof comprising one or more conservative amino acid substitutions.

The targeting peptide may comprise a targeting sequence and a linear DNA-binding component (e.g. a linear DNA-binding polycationic component), wherein the targeting sequence is SEQ ID NO: 1, SEQ ID NO: 6, SEQ ID NO: 7, or SEQ ID NO: 8, or a variant thereof comprising one or more conservative amino acid substitutions.

The targeting peptide may comprise at least 4, at least 5, at least 6, at least 7, at least 8, at least 9, at least 10, at least 11, at least 12, at least 13, or at least 14 amino acids. The targeting sequence may comprise 4-100, 5-70, 6-50, or 7-40 amino acids.

The targeting peptide may comprise at least 4, at least 5, at least 6, at least 7, or at least 8 contiguous amino acids of SEQ ID NO: 2 or a variant thereof comprising one or more conservative amino acid substitutions. The targeting peptide may be SEQ ID NO: 2 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 2. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 2.

The targeting peptide may comprise at least 6, at least 7, at least 8, at least 9, or at least 10 contiguous amino acids of SEQ ID NO: 3 or a variant thereof comprising one or more conservative amino acid substitutions. The targeting peptide may be SEQ ID NO: 3 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 3. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 3.

The targeting peptide may comprise at least 18, at least 19, at least 20, at least 21, at least 22, at least 23, at least 24, at least 25, or at least 26 contiguous amino acids of SEQ ID NO: 4 or a variant thereof comprising one or more conservative amino acid substitutions. The targeting peptide may be SEQ ID NO: 4 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:4. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:4.

The targeting peptide may comprise at least 32, at least 33, at least 34, at least 35, at least 36, at least 37, least 38, at least 39, or at least 40 contiguous amino acids of SEQ ID NO: 5 or a variant thereof comprising one or more conservative amino acid substitutions. The targeting peptide may be SEQ ID NO: 5 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:5. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:5.

The targeting peptide may comprise at least 4, at least 5, at least 6, at least 7, or at least 8 contiguous amino acids of SEQ ID NO: 9 or a variant thereof comprising one or more conservative amino acid substitutions. The targeting peptide may be SEQ ID NO: 9 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 9. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 9.

The targeting peptide may comprise at least 6, at least 7, or at least 8, at least 9, at least 10 contiguous amino acids of SEQ ID NO: 10 or a variant thereof comprising one or more conservative amino acid substitutions. The targeting peptide may be SEQ ID NO: 10 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 10. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 10.

The targeting peptide may comprise at least 8, at least 9, at least 10, at least 11, at least 12, at least 13, or at least 14 contiguous amino acids of SEQ ID NO: 11 or a variant thereof comprising one or more conservative amino acid substitutions. The targeting peptide may be SEQ ID NO: 11 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 11. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 11

The targeting peptide may comprise at least 20, at least 21, at least 22, at least 23, least 24, at least 25, at least 26, at least 27, at least 28, at least 29, or at least 30 contiguous amino acids of SEQ ID NO: 12 or a variant thereof comprising one or more conservative amino acid substitutions. The targeting peptide may be SEQ ID NO: 12 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 12. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 12.

The targeting peptide may comprise at least 34, at least 35, at least 36, at least 37, least 38, at least 39, at least 40, at least 41, at least 42, at least 43, or at least 44 contiguous amino acids of SEQ ID NO: 13 or a variant thereof comprising one or more conservative amino acid substitutions. The targeting peptide may be SEQ ID NO: 13 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 13. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 13.

The targeting peptide may comprise at least 18, at least 19, at least 20, at least 21, at least 22, at least 23, at least 24, at least 25, or at least 26 contiguous amino acids of SEQ ID NO: 14 or a variant thereof comprising one or more conservative amino acid substitutions. The targeting peptide may be SEQ ID NO: 14 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 14. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 14.

The targeting peptide may comprise at least 32, at least 33, at least 34, at least 35, at least 36, at least 37, at least 38, at least 39, or at least 40 contiguous amino acids of SEQ ID NO: 15 or a variant thereof comprising one or more conservative amino acid substitutions. The targeting peptide may be SEQ ID NO: 15 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 15. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 15.

The targeting peptide may comprise at least 4, at least 5, at least 6, at least 7, or at least 8 contiguous amino acids of SEQ ID NO: 16 or a variant thereof comprising one or more conservative amino acid substitutions. The targeting peptide may be SEQ ID NO: 16 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 16. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 16.

The targeting peptide may comprise at least 16, at least 17, at least 18, at least 19, at least 20, at least 21, at least 22, at least 23, or at least 24 contiguous amino acids of SEQ ID NO: 17 or a variant thereof comprising one or more conservative amino acid substitutions. The targeting peptide may be SEQ ID NO: 17 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 17. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 17.

The targeting peptide may comprise at least 30, at least 31, at least 32, at least 33, at least 34, at least 35, at least 36, at least 37, or at least 38 contiguous amino acids of SEQ ID NO: 18 or a variant thereof comprising one or more conservative amino acid substitutions. The targeting peptide may be SEQ ID NO: 18 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 18. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 18.

The targeting peptide may comprise at least 7, at least 8, at least 9, at least 10, at least 11, at least 12, or at least 13, contiguous amino acids of SEQ ID NO: 19 or a variant thereof comprising one or more conservative amino acid substitutions. The targeting peptide may be SEQ ID NO: 19 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 19. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 19.

The targeting peptide may comprise at least 9, at least 10, at least 11, at least 12, at least 13, at least 14, or at least 15 contiguous amino acids of SEQ ID NO: 20 or a variant thereof comprising one or more conservative amino acid substitutions. The targeting peptide may be SEQ ID NO: 20 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 20. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 20.

The targeting peptide may comprise at least 11, at least 12, at least 13, at least 14, at least 15, at least 16, at least 17, at least 18, or at least 19 contiguous amino acids of SEQ ID NO: 21 or a variant thereof comprising one or more conservative amino acid substitutions. The targeting peptide may be SEQ ID NO: 21 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 21. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 21.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 22 or a variant thereof comprising one or more conservative amino acid substitutions. The targeting peptide may be SEQ ID NO: 22 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 22. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 22.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 23 or a variant thereof comprising one or more conservative amino acid substitutions. The targeting peptide may be SEQ ID NO: 23 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 23. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 23.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 24 or a variant thereof comprising one or more conservative amino acid substitutions. The targeting peptide may be SEQ ID NO: 24 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 24. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 24.

The targeting peptide may comprise at least 34, least 35, at least 36, at least 37, least 38, at least 39, at least 40, at least 41, at least 42, at least 43, at least 44, or at least 45 contiguous amino acids of SEQ ID NO: 25 or a variant thereof comprising one or more conservative amino acid substitutions. The targeting peptide may be SEQ ID NO: 25 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 25. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 25.

The targeting peptide may comprise at least 6, at least 7, at least 8, at least 9, or at least 10, at least 11, at least 12, or at least 13 contiguous amino acids of SEQ ID NO: 26 or a variant thereof comprising one or more conservative amino acid substitutions. The targeting peptide may be SEQ ID NO: 26 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 26. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 26.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 27 or a variant thereof comprising one or more conservative amino acid substitutions. The targeting peptide may be SEQ ID NO: 27 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 27. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 27.

The targeting peptide may comprise at least 33, least 34, at least 35, least 36, at least 37, at least 38, least 39, at least 40, at least 41, at least 42, at least 43, or at least 44 contiguous amino acids of SEQ ID NO: 28 or a variant thereof comprising one or more conservative amino acid substitutions. The targeting peptide may be SEQ ID NO: 28 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 28. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 28.

The targeting peptide may comprise at least 6, at least 7, at least 8, at least 9, or at least 10, at least 11, at least 12, or at least 13 contiguous amino acids of SEQ ID NO: 29 or a variant thereof comprising one or more conservative amino acid substitutions. The targeting peptide may be SEQ ID NO: 29 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 29. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 29.

The targeting peptide may comprise at least 8, at least 9, or at least 10, at least 11, at least 12, at least 13, at least 14, or at least 15 contiguous amino acids of SEQ ID NO: 30 or a variant thereof comprising one or more conservative amino acid substitutions. The targeting peptide may be SEQ ID NO: 30 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 30. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 30.

The targeting peptide may comprise at least 11, at least 12, at least 13, at least 14, at least 15, at least 16, at least 17, at least 18, or at least 19 contiguous amino acids of SEQ ID NO: 31 or a variant thereof comprising one or more conservative amino acid substitutions. The targeting peptide may be SEQ ID NO: 31 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 31. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 31.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 32 or a variant thereof comprising one or more conservative amino acid substitutions. The targeting peptide may be SEQ ID NO: 32 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 32. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 32.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 33 or a variant thereof comprising one or more conservative amino acid substitutions. The targeting peptide may be SEQ ID NO: 33 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 33. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 33.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 34 or a variant thereof comprising one or more conservative amino acid substitutions. The targeting peptide may be SEQ ID NO: 34 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 34. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 34.

The targeting peptide may comprise at least 35, at least 36, at least 37, least 38, at least 39, at least 40, at least 41, at least 42, at least 43, at least 44, or at least 45 contiguous amino acids of SEQ ID NO: 35 or a variant thereof comprising one or more conservative amino acid substitutions. The targeting peptide may be SEQ ID NO: 35 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 35. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 35.

The targeting peptide may comprise at least 6, at least 7, at least 8, at least 9, or at least 10, at least 11, at least 12, or at least 13 contiguous amino acids of SEQ ID NO: 36 or a variant thereof comprising one or more conservative amino acid substitutions. The targeting peptide may be SEQ ID NO: 36 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 36. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 36.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 37 or a variant thereof comprising one or more conservative amino acid substitutions. The targeting peptide may be SEQ ID NO: 37 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 37. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 37.

The targeting peptide may comprise at least 32, least 33, at least 34, least 35, at least 36, at least 37, least 38, at least 39, at least 40, at least 41, at least 42, or at least 43 contiguous amino acids of SEQ ID NO: 38 or a variant thereof comprising one or more conservative amino acid substitutions. The targeting peptide may be SEQ ID NO: 38 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 38. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 38.

By "sequence identity" or "sequence similarity" is meant that the identity or similarity, respectively, between two or more amino acid sequences, or two or more nucleotide sequences, is expressed in terms of the identity or similarity between the sequences. Sequence identity can be measured in terms of "percentage (%) identity," in which a higher percentage indicates greater identity shared between the sequences. Sequence similarity can be measured in terms of percentage similarity (which takes into account conservative amino acid substitutions); the higher the percentage, the more similarity shared between the sequences.

The targeting peptides described herein may comprise conservative amino acid substitutions at one or more amino acid residues, e.g. at essential or non-essential amino acid residues. A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagines, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The targeting peptide may comprise a cyclic region, a branched region, and/or a linear region.

The targeting peptide comprising the cyclic region may be formed by the provision of at least two cysteine residues in the peptide, thus enabling the formation of a disulphide bond. Thus, the targeting peptide may comprise two or more cysteine residues that are capable of forming one or more disulphide bond(s). Preferably, the two or more cysteine residues flank the targeting sequence. For example, if the targeting sequence is ASSLNIA (SEQ ID NO: 6), the targeting peptide may comprise a sequence: CASSLNIAC (SEQ ID NO: 9).

The targeting peptide may comprise a linker. The linker may be cleavable or non-cleavable.

The invention provides a targeting peptide comprising:
(a) a targeting sequence; and
(b) a linker.

The invention provides a targeting peptide comprising:
(a) a targeting sequence;
(b) a linker; and
(c) a nucleic acid-binding component.

The linker may comprise at least 1, at least 2, at least 3, at least 4, at least 5, at least 6, at least 7, at least 8, at least 9, at least 10, at least 11 or at least 12 amino acids. The linker may comprise 2-10 amino acids or 4-8 amino acids. The amino acids may be naturally occurring or non-naturally occurring. They may have L- or D- configuration. The amino acids may be the same or different. The use of multiple lysine residues (or other cationic amino acids suitable for use in the nucleic acid-binding polycationic component) should generally be avoided in the linker as oligo-lysine sequences have activity as a nucleic acid-binding polycationic component.

The linker may comprise a cleavable portion that is susceptible to cleavage within a cell. The linker that comprises a cleavable portion that is susceptible to cleavage within a cell may be susceptible to cleavage within the endosome, lysosome, and/or cytoplasm of a cell. The expression "susceptible to cleavage" refers to a linker that is susceptible to cleavage over a timescale during which the remaining elements on the targeting peptide are intact. Thus, the linker may be cleaved more rapidly than the cellular peptide-degradation pathways take effect. The cleavable portion may comprise from 3 to 6 amino acids, for example 4 amino acids. The linker may include the amino acid sequence RVRR (SEQ ID NO: 39) as a cleavable portion. The amino acid sequence RVRR is susceptible to enzymatic cleavage by the endosomal protease furin. The cleavable portion of the linker may be attached to a nucleic acid-binding component. The cleavable portion of the linker may be cleavable by a protease. The protease may be cathepsin (e.g. serine, cysteine, aspartic-type), furin, a lysosomal protease or an endosomal protease.

The targeting peptide may comprise a spacer. The spacer may be either a peptide, that is to say, it comprises amino acid residues, or a polyethyleneglycol group, or a mixture of the two. The amino acids may be naturally occurring or non-naturally occurring. They may have L- or D-configuration. The spacer may have one or more amino acids. The spacer may comprise at least 1, at least 2, at least 3, at least 4, at least 5, at least 6, at least 7, at least 8, at least 9, at least 10, at least 11 or at least 12 amino acids.

The spacer may comprise 2-7 amino acids, preferably 2-5 amino acids. The amino acids may be the same or different. The spacer may comprise the dipeptide glycine-glycine (GG), glycine-alanine (GA) or alanine-alanine (AA). The spacer may comprise a hydrophobic spacer. The spacer may include the amino acid sequence XSX in which X is c-aminocaproic acid (c-Ahx) also known as 6-aminohexanoic acid, a synthetic, i.e. non-naturally occurring, amino acid. Aminocaproic acid functions as a hydrophobic spacer. The spacer may comprise GG, GA, AA, XSXGG (SEQ ID NO: 40), XSXGA (SEQ ID NO: 41) or XSXAA (SEQ ID NO: 43). Preferably, the spacer comprises GA or GG. The spacer may be located at the end of the linker in a targeting peptide. The spacer may be attached to a targeting sequence or a targeting sequence flanked by cysteine residues. Preferably, the spacer links the linker and the targeting sequence (which is optionally flanked by the cysteine residues).

Thus, the invention provides a targeting peptide comprising:
(a) a targeting sequence;
(b) a linker; and
(c) a spacer.

Thus, the invention provides a targeting peptide comprising:
(a) a targeting sequence;
(b) a linker; and
(c) a nucleic acid-binding component.

The invention provides a targeting peptide comprising:
(a) a targeting sequence;
(b) a linker
(c) a spacer; and
(d) a nucleic acid-binding component.

The targeting peptide may have a structure: A-B-C-D, wherein component A is a nucleic acid-binding component, component B is a linker, component C is a spacer and component D is a targeting sequence (optionally flanked by the cysteine residues).

The targeting peptide may have a structure: A-B-D, wherein component A is a nucleic acid-binding component, component B is a linker and component D is a targeting sequence (optionally flanked by the cysteine residues).

The targeting peptide may have a structure: A-D, wherein component A is a nucleic acid-binding component and component D is a targeting sequence (optionally flanked by the cysteine residues).

The nucleic acid-binding component may be a nucleic acid-binding cationic component or a nucleic acid-binding neutral component. The cationic component and/or the neutral component may be used to establish a desired charge (i.e. a negative/positive ratio) of the nanoparticle. A specific charge (i.e. a Nitrogen/Phosphate ratio) may be required to facilitate or enhance cell transfection.

The nucleic acid-binding component may be a DNA-binding component. The DNA-binding component may be a DNA-binding cationic component, or a DNA-binding neutral component. The nucleic acid-binding component may be a closed linear DNA-binding component. The closed linear DNA-binding component may be a closed linear DNA-binding cationic component, or a closed linear DNA-binding neutral component. The nucleic acid-binding component may be a linear DNA-binding component (e.g. linear double-stranded DNA-binding component). The closed linear DNA-binding component may be a closed linear DNA-binding cationic component (e.g. linear double-stranded DNA-binding cationic component), or a closed linear DNA-binding neutral component (e.g. linear double-stranded DNA-binding neutral component).

Preferably, the nucleic acid-binding component is a nucleic acid-binding cationic component (e.g. DNA-binding cationic component, closed linear DNA-binding cationic component, or linear DNA-binding cationic component).

The nucleic acid-binding cationic component may be a nucleic acid-binding polycationic component, The nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the nucleic acid-binding polycationic component comprises at least 16, at least 17 or at least 30 cationic monomers.

The nucleic acid-binding cationic component may comprise a lysine, a histidine, or an arginine. The nucleic acid- binding polycationic component may comprise a lysine, a histidine, or an arginine. The nucleic acid-binding polycationic component may comprise an oligolysine (linear or branched), an oligohistidine (linear or branched) or an oligoarginine (linear or branched). For example, the nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the nucleic acid-binding polycationic component comprises at least 16, at least 17, or at least 30 lysine residues. More preferably still, the nucleic acid-binding polycationic component comprises at least 17 lysine residues.

The nucleic acid-binding neutral component may be a nucleic acid-binding polyneutral component, The nucleic acid-binding polyneutral component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 neutral monomers.

The closed linear DNA-binding cationic component may be a closed linear DNA-binding polycationic component, The closed linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the closed linear DNA-binding polycationic component comprises at least 16, at least 17 or at least 30 cationic monomers.

The closed linear DNA-binding cationic component may comprise a lysine, a histidine, or an arginine. The closed linear DNA-binding polycationic component may comprise a lysine, a histidine, or an arginine. The closed linear DNA-binding polycationic component may comprise an oligolysine (linear or branched), an oligohistidine (linear or branched) or an oligoarginine (linear or branched). For example, the closed linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the closed linear DNA-binding polycationic component comprises at least 16, at least 17, or at least 30 lysine residues. More preferably still, the closed linear DNA-binding polycationic component comprises at least 17 lysine residues.

The linear DNA-binding cationic component may be a linear DNA-binding polycationic component, The linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the linear DNA-binding polycationic component comprises at least 16, at least 17 or at least 30 cationic monomers.

The linear DNA-binding cationic component may comprise a lysine, a histidine, or an arginine. The linear DNA-binding polycationic component may comprise a lysine, a histidine, or an arginine. The linear DNA-binding polycationic component may comprise an oligolysine (linear or branched), an oligohistidine (linear or branched) or an oligoarginine (linear or branched). For example, the linear DNA-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the linear DNA-binding polycationic component comprises at least 16, at least 17, or at least 30 lysine residues. More preferably still, the linear DNA-binding polycationic component comprises at least 17 lysine residues.

The nucleic acid-binding component may be linear or branched. The nucleic acid-binding component may be a nucleic acid-binding polycationic component or a nucleic acid-binding polyneutral component. The nucleic acid-binding polycationic component may be linear or branched. The nucleic acid-binding polyneutral component may be linear or branched. For example, the nucleic acid-binding polycationic component may comprise at least 16, at least 17, or at least 30 lysine residues in a linear chain. Alternatively, the nucleic acid-binding polycationic component may comprise at least 16, at least 17, or at least 30 lysine residues in a branched chain.

Thus, the targeting peptide may comprise a targeting sequence and a nucleic acid-binding component. The targeting peptide may comprise a targeting sequence and a nucleic acid-binding cationic component. The targeting peptide may comprise a targeting sequence and a nucleic acid-binding polycationic component (e.g. DNA-binding polycationic component). The targeting peptide may comprise a targeting sequence and a closed linear DNA-binding component (e.g. a closed linear DNA-binding polycationic component). The targeting peptide may comprise a targeting sequence and a linear DNA-binding component (e.g. a linear DNA-binding polycationic component).

The targeting peptide may comprise:
(a) a targeting sequence;
(b) a spacer; and
(c) a nucleic acid-binding component.

The targeting peptide may comprise a targeting sequence, a spacer, and a nucleic acid-binding cationic component. The targeting peptide may comprise a targeting sequence, a spacer, and a nucleic acid-binding polycationic component. The targeting peptide may comprise a targeting sequence, a spacer, and a closed linear DNA-binding component (e.g. a closed linear DNA-binding polycationic component). The targeting peptide may comprise a targeting sequence, a spacer, and a linear DNA-binding component (e.g. a linear DNA-binding polycationic component).

The targeting peptide may comprise:
(a) a targeting sequence;
(b) a linker; and
(c) a nucleic acid-binding component.

The targeting peptide may comprise a targeting sequence, a linker, and a nucleic acid-binding cationic component. The targeting peptide may comprise a targeting sequence, a linker, and a nucleic acid-binding polycationic component. The targeting peptide may comprise a targeting sequence, a linker, and a closed linear DNA-binding component (e.g. a closed linear DNA-binding polycationic component). The targeting peptide may comprise a targeting sequence, a linker, and a linear DNA-binding component (e.g. a linear DNA-binding polycationic component).

The targeting peptide may comprise:
(a) a targeting sequence;
(b) a linker
(c) a spacer; and
(d) a nucleic acid-binding component.

The targeting peptide may comprise a targeting sequence, a linker, a spacer, and a nucleic acid-binding cationic component. The targeting peptide may comprise a targeting sequence, a linker, a spacer, and a nucleic acid-binding polycationic component. The targeting peptide may comprise a targeting sequence, a linker, a spacer, and a closed linear DNA-binding component (e.g. a closed linear DNA-binding polycationic component). The targeting peptide may comprise a targeting sequence, a linker, a spacer, and a linear DNA-binding component (e.g. a linear DNA-binding polycationic component).

Preferably, the targeting sequence is SEQ ID NO: 1, SEQ ID NO: 6, SEQ ID NO: 7, or SEQ ID NO: 8. Thus, the targeting peptide may comprise:
(a) a targeting sequence, wherein the targeting sequence is: SEQ ID NO: 1, SEQ ID NO: 6, SEQ ID NO: 7, or SEQ ID NO: 8; and
(b) a nucleic acid-binding component (e.g. a DNA-binding polycationic component).

The targeting peptide may comprise:
(a) a targeting sequence, wherein the targeting sequence is: SEQ ID NO: 1, SEQ ID NO: 6, SEQ ID NO: 7, or SEQ ID NO: 8;
(b) a spacer; and
(c) a nucleic acid-binding component (e.g. a DNA-binding polycationic component).

The targeting peptide may comprise:
(a) a targeting sequence, wherein the targeting sequence is: SEQ ID NO: 1, SEQ ID NO: 6, SEQ ID NO: 7, or SEQ ID NO: 8;
(b) a linker; and
(c) a nucleic acid-binding component (e.g. a DNA-binding polycationic component).

The targeting peptide may comprise:
(a) a targeting sequence, wherein the targeting sequence is: SEQ ID NO: 1, SEQ ID NO: 6, SEQ ID NO: 7, or SEQ ID NO: 8;
(b) a linker;
(c) a spacer; and
(d) a nucleic acid-binding component (e.g. a DNA-binding polycationic component).

The targeting peptide may comprise:
(a) a targeting sequence, wherein the targeting sequence is: SEQ ID NO: 1, SEQ ID NO: 6, SEQ ID NO: 7, or SEQ ID NO: 8; and
(b) a spacer.

The targeting peptide may comprise:
(a) a targeting sequence, wherein the targeting sequence is: SEQ ID NO: 1, SEQ ID NO: 6, SEQ ID NO: 7, or SEQ ID NO: 8; and
(b) a linker.

The targeting peptide may comprise:
(a) a targeting sequence, wherein the targeting sequence is: SEQ ID NO: 1, SEQ ID NO: 6, SEQ ID NO: 7, or SEQ ID NO: 8;
(b) a linker; and
(c) a spacer.

The targeting sequence may target a cell-surface receptor or a biologically active molecule present on the surface of a cell. The targeting sequence may target a brain cell, a kidney cell, a muscle cell, a skin cell (e.g. a skin epithelial cell) or a spleen cell. The targeting sequence may be a brain cell targeting sequence. The targeting sequence may target a cell-surface receptor or a biologically active molecule present on the surface of brain cells. The targeting sequence may be a kidney cell targeting sequence. The targeting sequence may target a cell-surface receptor or a biologically active molecule present on the surface of kidney cells. The targeting sequence may be a muscle cell targeting sequence. The targeting sequence may target a cell-surface receptor or a biologically active molecule present on the surface of muscle cells. The targeting sequence may be a skin cell (e.g. a skin epithelial cell) targeting sequence. The targeting sequence may target a cell-surface receptor or a biologically active molecule present on the surface of skin cells (e.g. skin epithelial cells). The targeting sequence may be a spleen cell targeting sequence. The targeting sequence may target a cell-surface receptor or a biologically active molecule present on the surface of spleen cells.

The term "brain cell targeting sequence" refers to a sequence that has the ability to target, bind and/or interact with a brain cell. For example, the brain cell targeting sequence may target, bind and/or interact with a receptor on a muscle cell, or a biologically active molecule present on the surface of brain cells. The term "kidney cell targeting sequence" refers to a sequence that has the ability to target, bind and/or interact with a kidney cell. For example, the kidney cell targeting sequence may target, bind and/or interact with a receptor on a kidney cell, or a biologically active molecule present on the surface of kidney cells. The term "muscle cell targeting sequence" refers to a sequence that has the ability to target, bind and/or interact with a muscle cell. For example, the muscle cell targeting sequence may target, bind and/or interact with a receptor on a muscle cell, or a biologically active molecule present on the surface of muscle cells. The term "skin cell targeting sequence" refers to a sequence that has the ability to target, bind and/or interact with a skin cell. For example, the skin cell targeting sequence may target, bind and/or interact with a receptor on a skin cell, or a biologically active molecule present on the surface of skin cells. The term "spleen cell targeting sequence" refers to a sequence that has the ability to target, bind and/or interact with a spleen cell. For example, the spleen cell targeting sequence may target, bind and/or interact with a receptor on a spleen cell, or a biologically active molecule present on the surface of spleen cells.

The targeting sequence may be a cell type-specific targeting sequence (e.g. a brain-specific targeting sequence, a kidney-specific targeting sequence, a muscle-specific targeting sequence, a skin-specific targeting sequence or a spleen-specific targeting sequence). The term "specific" refers to the ability to preferentially target, bind and/or interact with a given target, for example, a receptor on a brain, kidney, muscle, skin or spleen cell, over other targets. Preferential targeting, binding and/or interacting with may be assessed by measuring the binding affinity of a sequence for a target receptor on a target cell in comparison to a target on a different cell. Similarly, preferential targeting, binding and/or interacting with may be measured by calculating a dissociation rate (of a sequence from a target molecule). Preferential targeting may be assessed by comparing the amount of binding events for each one of the target cells. For example, the brain-specific targeting sequence may bind to a brain cell more often than any other cell type. The brain-specific targeting sequence may bind with higher affinity for a brain cell (or a receptor on a brain cell) than any other cell (or receptor) type. For example, the kidney-specific targeting sequence may bind to a kidney cell more often than any other cell type. The kidney-specific targeting sequence may bind with higher affinity for a kidney cell (or a receptor on a kidney cell) than any other cell (or receptor) type. For example, the muscle-specific targeting sequence may bind to a muscle cell more often than any other cell type. The muscle-specific targeting sequence may bind with higher affinity for a muscle cell (or a receptor on a muscle cell) than any other cell (or receptor) type. For example, the skin-specific targeting sequence may bind to a skin cell more often than any other cell type. The skin-specific targeting sequence may bind with higher affinity for a skin cell (or a receptor on a skin cell) than any other cell (or receptor) type. For example, the spleen-specific targeting sequence may bind to a spleen cell more often than any other cell type. The spleen-specific targeting sequence may bind with higher affinity for a spleen cell (or a receptor on a spleen cell) than any other cell (or receptor) type.

The targeting peptide may comprise:
(a) a targeting sequence, wherein the targeting sequence is a brain cell targeting sequence;
(b) a linker; and
(c) a spacer.

The targeting peptide may comprise:
(a) a targeting sequence, wherein the targeting sequence is a brain cell targeting sequence;
(b) a linker; and
(c) a nucleic acid-binding component.

The targeting peptide may comprise:
(a) a targeting sequence, wherein the targeting sequence is a brain cell targeting sequence;
(b) a linker
(c) a spacer; and
(d) a nucleic acid-binding component.

The targeting peptide may comprise:
(a) a targeting sequence, wherein the targeting sequence is an brain cell targeting sequence; and
(b) a nucleic-acid binding component.

The targeting peptide may comprise:
(a) a targeting sequence, wherein the targeting sequence is a kidney cell targeting sequence; and
(b) a nucleic-acid binding component.

The targeting peptide may comprise:
(a) a targeting sequence, wherein the targeting sequence is a kidney cell targeting sequence;
(b) a linker; and
(c) a spacer.

The targeting peptide may comprise:
(a) a targeting sequence, wherein the targeting sequence is a kidney cell targeting sequence;
(b) a linker; and
(c) a nucleic acid-binding component.

The targeting peptide may comprise:
(a) a targeting sequence, wherein the targeting sequence is a kidney cell targeting sequence;
(b) a linker
(c) a spacer; and
(d) a nucleic acid-binding component.

The targeting peptide may comprise:
(a) a targeting sequence, wherein the targeting sequence is a muscle cell targeting sequence; and
(b) a nucleic-acid binding component.

The targeting peptide may comprise:
(c) a targeting sequence, wherein the targeting sequence is a muscle cell targeting sequence;
(d) a linker; and
(e) a spacer.

The targeting peptide may comprise:
(a) a targeting sequence, wherein the targeting sequence is a muscle cell targeting sequence;
(b) a linker; and
(c) a nucleic acid-binding component.

The targeting peptide may comprise:
(a) a targeting sequence, wherein the targeting sequence is a muscle cell targeting sequence;
(b) a linker
(c) a spacer; and
(d) a nucleic acid-binding component.

The targeting peptide may comprise:
(a) a targeting sequence, wherein the targeting sequence is a skin cell targeting sequence; and
(b) a nucleic-acid binding component.

The targeting peptide may comprise:
(a) a targeting sequence, wherein the targeting sequence is a skin cell targeting sequence;
(b) a linker; and
(c) a spacer.

The targeting peptide may comprise:
(a) a targeting sequence, wherein the targeting sequence is a skin cell targeting sequence;
(b) a linker; and
(c) a nucleic acid-binding component.

The targeting peptide may comprise:
(a) a targeting sequence, wherein the targeting sequence is a skin cell targeting sequence;
(b) a linker
(c) a spacer; and
(d) a nucleic acid-binding component.

The targeting peptide may comprise:
(a) a targeting sequence, wherein the targeting sequence is a spleen cell targeting sequence; and
(b) a nucleic-acid binding component.

The targeting peptide may comprise:
(a) a targeting sequence, wherein the targeting sequence is a spleen cell targeting sequence;
(b) a linker; and
(c) a spacer.

The targeting peptide may comprise:
(a) a targeting sequence, wherein the targeting sequence is a spleen cell targeting sequence;
(b) a linker; and
(c) a nucleic acid-binding component.

The targeting peptide may comprise:
(a) a targeting sequence, wherein the targeting sequence is a spleen cell targeting sequence;
(b) a linker
(c) a spacer; and
(d) a nucleic acid-binding component.

### 3. Nucleic acid cargos

The cargo suitable for use in the nanoparticle (e.g. non-viral transfection complex) described herein is a nucleic acid. Preferably, the cargo is a closed linear DNA molecule or a linear DNA molecule. The closed linear DNA molecule and/or the linear DNA molecule may have an enhanced resistance to nuclease (e.g. exonuclease) digestion.

The nucleic acid may be a DNA molecule. The nucleic acid may be a closed linear DNA molecule (e.g. a covalently-closed linear DNA molecule) or a linear DNA molecule (e.g. linear double-stranded DNA molecule). The DNA molecule may be a linear DNA molecule or a partially linear DNA molecule (i.e. the DNA molecule may comprise a linear portion). The nucleic acid may be single-stranded, double-stranded, or partially single-stranded and partially double-stranded.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid (e.g. the closed linear DNA molecule) comprises at least 500 nucleotides.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid (e.g. the closed linear DNA molecule) comprises at least 500 base pairs.

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The closed linear DNA molecule has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open-ended cassettes have been demonstrated to be inefficient with respect to gene expression when introduced into host tissue. This has been attributed to cassette instability due to the action of exonucleases in the extracellular space.

Sequestering DNA ends inside closed structures also has other advantages. The DNA ends are prevented from integrating with genomic DNA and so closed linear DNA molecules offer improved safety. In addition, the closed linear structure reduces concatamerisation of DNA molecules inside host cells and thus expression levels of the gene product can be regulated in a more sensitive manner.

The closed linear DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The closed linear DNA molecule may comprise a spacer region. The spacer region may be at least 10, at least 20, at least 30, at least 40, at least 50, at least 60, at least 70, at least 80, at least 90, at least 100, at least 125, at least 150, at least 175, or at least 200 base pairs.

The closed linear DNA molecule may comprise an inverted terminal repeat sequence.

The closed linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the closed linear DNA molecule comprises at least 500 base pairs.

The closed linear DNA molecule may comprise one or more protected nucleotides (i.e. nucleotides resistant to nuclease (e.g. exonuclease) digestion). The closed linear DNA molecule may comprise one or more of protected nucleotides (i.e. nucleotides resistant to nuclease (e.g. exonuclease) in each strand. For example, the closed linear DNA molecule may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) in each strand.

The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The protected nucleotides may be located at the 3' and/or 5' end and/or 3' and/or 5' end region of the linear double-stranded DNA molecule. Preferably, the protected nucleotides are located at the 3' and 5' end and at the 3' and 5' region of the linear double-stranded DNA molecule. The 3' region comprises at least 20 3'-end nucleotides of the linear DNA molecule. The 3' region comprises less than 30 3'-end nucleotides of the linear DNA molecule. The 5' region comprises no more than 30 5'-end nucleotides of the linear DNA molecule. That is to say that the nucleotides which are the last and/or the first nucleotides in the linear DNA molecule may be protected nucleotides. In other words, the linear DNA molecule may comprise a "cap" of protected nucleotides, which protects the ends of the linear DNA molecule from nuclease (e.g. exonuclease) digestion.

The protected nucleotides (i.e. nucleotides resistant to nuclease (e.g. exonuclease) digestion) may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The protected nucleotides (i.e. nucleotides resistant to nuclease (e.g. exonuclease) digestion) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The linear double-stranded DNA molecule may comprise one or more protected nucleotides (e.g. phosphorothioated nucleotides) at internal positions in each strand (i.e. the sense and antisense strands).

The internal positions may be any positions in the linear double-stranded DNA molecule other than the last nucleotide on the 3'-end and the 5'-end of the sense and antisense strands. The internal positions may be located at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule. The internal positions may be located at least 7 nucleotides away from the 3'-end and/or the 5'-end of each strand of the linear double-stranded DNA molecule. Preferably, the internal positions are located at least 10 nucleotides away from the 3'-end and/or the 5'-end of each strand of the linear double-stranded DNA molecule. In the linear double-stranded DNA molecule, the internal positions may be located at least 7 nucleotides away from the 3'-end and the 5'-end of the sense strand. Preferably, in the linear double-stranded DNA molecule, the internal positions are located at least 10 nucleotides away from the 3'-end and the 5'-end of the sense strand. In the linear double-stranded DNA molecule, the internal positions may be located at least 7 nucleotides away from the 3'-end and the 5'-end of the antisense strand. Preferably, in the linear double-stranded DNA molecule, the internal positions may be located at least 10 nucleotides away from the 3'-end and the 5'-end of the antisense strand. In the linear double-stranded DNA molecule, the internal positions may be located at least 7 nucleotides away from the 3'-end of each strand. Preferably, in the linear double-stranded DNA molecule, the internal positions may be located at least 10 nucleotides away from the 3'-end of each strand. In the linear double-stranded DNA molecule, the internal positions may be located at least 7 nucleotides away from the 5'-end of each strand. Preferably, in the linear double-stranded DNA molecule, the internal positions may be located at least 10 nucleotides away from the 5'-end of each strand.

The linear double-stranded DNA molecule may comprise a cassette. The location of the protected (e.g. phosphorothioated) nucleotides in the linear double-stranded DNA molecule may be such that the cassette is protected from nuclease (e.g. exonuclease III) digestion. Thus, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette

The term *"5'-end nucleotide of the cassette"* as used herein is intended to encompass the 5'-end nucleotide of each strand of the cassette. Thus, in the linear double-stranded DNA molecule, the cassette typically comprises a 5'-end nucleotide of the sense strand and a 5'-end nucleotide of the antisense strand.

The term *"3'-end nucleotide of the cassette"* as used herein is intended to encompass the 3'-end nucleotide of each strand of the cassette. Thus, in the linear double-stranded DNA molecule, the cassette typically comprises a 3'-end nucleotide of the sense strand and a 3'-end nucleotide of the antisense strand.

The term *"outside of the cassette"* as used herein is intended to encompass any nucleotides which are not part of the cassette. This includes any nucleotides that are comprised in the linear double-stranded DNA molecule and which also do not form part of the cassette. The term *"N nucleotides outside of the cassette"* or *"N nucleotides away from the cassette"* is intended to describe nucleotides that are located N nucleotides from the end of the cassette towards the end of the linear double-stranded DNA molecule. For example, the term *"2 nucleotides outside of the cassette"* in the context of internal positions of nucleotides is meant to describe a nucleotide that is outside of the cassette and that is two nucleotides away from the last nucleotide of the cassette. For example, in the sequence: 5'-AAAAAACATAAAA (SEQ ID NO: 42), wherein the cassette starts from nucleotide "T" (in the 5' to 3' direction), the term *"2 nucleotides outside of the cassette"* refers to the "C" nucleotide. Thus, the term *"at least 2 nucleotides outside of the cassette"* or *"at least 2 nucleotides away from the cassette"* is meant to describe nucleotides that are outside of the cassette and that are at least two nucleotides away from the last nucleotide of the cassette. In the example above, nucleotides *"at least 2 nucleotides away from the cassette"* would be any nucleotides selected from 5'-AAAAAAC. Similarly, the term *"at least 2 nucleotides away from the 5'-end of the cassette"* is meant to describe nucleotides that are outside of the cassette and that are at least two nucleotides away from the last nucleotide at the 5'-end of the cassette. In the example above, the last nucleotide at the 5'-end of the cassette is "T", and nucleotides *"at least 2 nucleotides away from the 5'-end of the cassette"* would be any nucleotides selected from 5'-AAAAAAC.

The internal positions may not be located between the second and penultimate nucleotide of the cassette. The internal positions may be any position in the linear double-stranded DNA molecule other than the last nucleotide on the 3'-end and the 5'-end of the sense and antisense strands and other than nucleotides located between the second and penultimate nucleotide of the cassette. The internal positions may be located outside of the cassette and at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule and/or at least 2, 3, 4, 5, 6, 7, 8, 9, 10, 12, 14, 16, 18, 20, 30, 40, 50, 75, or 100 nucleotides away (i.e. outside of the cassette) from the 3'-end and/or the 5'-end of each strand of the cassette. Preferably, the internal positions is located outside of the cassette and at least 6, at least 8, or at least 10 nucleotides away from the 3'-end and/or the 5'-end of each strand of the linear double-stranded DNA molecule and/or at least 6, at least 8, or at least 10 nucleotides away from the 3'-end and/or the 5'-end of each strand of the cassette (i.e. at least 6, at least 8, or at least 10 nucleotides outside of the cassette). Preferably, the cassette does not comprise a phosphorothioated nucleotide at either one of the positions 1-2, 1-3, 1-4, 1-5, 1-6, 1-7, 1-8, 1-9, 1-10, 1-12, 1-14, 1-16, 1-18, or 1-20 of the sense and/or the antisense strand away from the 3'-end and/or the 5'-end of the cassette (i.e. outside of the cassette). Preferably, the internal positions in each strand are located at least 6 nucleotides away from the end of the linear double-stranded DNA molecule and are not located between the second and penultimate nucleotide of the cassette. Preferably, the internal positions in each strand are located at least 10 nucleotides away from the end of the linear double-stranded DNA molecule and are not located between the second and penultimate nucleotide of the cassette. Preferably, the internal positions in each strand are located at least 6 nucleotides away from the end of the linear double-stranded DNA molecule and at least 6 nucleotides away from the end of the cassette (i.e. at least 6 nucleotides outside of the cassette). Preferably, the internal positions in each strand are located at least 10 nucleotides away from the end of the linear double-stranded DNA molecule and at least 10 nucleotides away from the end of the cassette (i.e. at least 10 nucleotides outside of the cassette). The linear double-stranded DNA molecule may comprise a first phosphorothioated nucleotide at an internal position which is a position other than the last nucleotide on the 3'-end and the 5'-end of the sense and antisense strands as long as this position is located at least 2, 3, 4, 5, 6, 7, 8, 9, 10, 12, 14, 16, 18, 20, 30, 40, or 50 nucleotides outside of the cassette. For example, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are not located between the second and penultimate nucleotide of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located outside of the cassette. The linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 2, 3, 4, 5, 6, 7, 8, 9, 10, 12, 14, 16, 18, 20, 30, 40, or 50 nucleotides outside of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 6, 8, or 10 nucleotides outside of the cassette.

The linear double-stranded DNA molecule may comprise at least 2, 3, 4, 5, 6, 7, 8, 9 or 10 protected (e.g. phosphorothioated) nucleotides at internal positions in each strand. Preferably, the linear double-stranded DNA molecule comprises at least 2 protected (e.g. phosphorothioated) nucleotides at internal positions in each strand.

The location of phosphorothioated nucleotides in the sense strand of the linear double-stranded DNA molecule may be such that the one or more phosphorothioated nucleotides are located upstream of the cassette (i.e. towards the 5'-end of the sense strand of the DNA molecule).

In the sense strand of the linear double-stranded DNA molecule:
(a) one of the at least two phosphorothioated nucleotides may be the 5'-end nucleotide of the cassette; and/or
(b) one of the at least two phosphorothioated nucleotides may be in a first region of the sense strand,
wherein the first region of the sense strand is 5' of the cassette.

The location of phosphorothioated nucleotides in the sense strand of the linear double-stranded DNA molecule may be such that the one or more phosphorothioated nucleotides are located downstream of the cassette (i.e. towards the 3'-end of the sense strand of the DNA molecule).

In the sense strand of the linear double-stranded DNA molecule:
(a) one of the at least two phosphorothioated nucleotides may be the 3'-end nucleotide of the cassette; and/or
(b) one of the at least two phosphorothioated nucleotides may be in a second region of the sense strand, wherein the second region of the sense strand is 3' of the cassette.

The location of phosphorothioated nucleotides in the antisense strand of the linear double-stranded DNA molecule may be such that the one or more phosphorothioated nucleotides are located upstream of the cassette (i.e. towards the 5'-end of the antisense strand of the DNA molecule).

In the antisense strand of the linear double-stranded DNA molecule:
(a) one of the at least two phosphorothioated nucleotides may be the 5'-end nucleotide of the cassette; and/or
(b) one of the at least two phosphorothioated nucleotides may be in a first region of the antisense strand, wherein the first region of the antisense strand is 5' of the cassette.

The location of phosphorothioated nucleotides in the antisense strand of the linear double-stranded DNA molecule may be such that the one or more phosphorothioated nucleotides are located downstream of the cassette (i.e. towards the 3'-end of the antisense strand of the DNA molecule).

In the antisense strand of the linear double-stranded DNA molecule:
(a) one of the at least two phosphorothioated nucleotides may be the 3'-end nucleotide of the cassette; and/or
(b) one of the at least two phosphorothioated nucleotides may be in a second region of the antisense strand, wherein the second region of the antisense strand is 3' of the cassette.

The location of phosphorothioated nucleotides in both the sense and the antisense strand of the linear double-stranded DNA molecule may be such that at least one phosphorothioated nucleotide is located downstream of the cassette and at least one phosphorothioated nucleotide is located upstream of the cassette in each strand.

In the linear double-stranded DNA molecule:
(a) in the sense strand one of the at least two phosphorothioated nucleotides may be the 5'-end nucleotide of the cassette, and/or one of the at least two phosphorothioated nucleotides may be in a first region of the sense strand, wherein the first region of the sense strand is 5' of the cassette;
(b) in the sense strand one of the at least two phosphorothioated nucleotides may be the 3'-end nucleotide of the cassette, and/or one of the at least two phosphorothioated nucleotides may be in a second region of the sense strand, wherein the second region of the sense strand is 3' of the cassette;
(c) in the antisense strand one of the at least two phosphorothioated nucleotides may be the 5'-end nucleotide of the cassette, and/or one of the at least two phosphorothioated nucleotides may be in a first region of the antisense strand, wherein the first region of the antisense strand is 5' of the cassette; and
(d) in the antisense strand one of the at least two phosphorothioated nucleotides may be the 3'-end nucleotide of the cassette, and/or one of the at least two phosphorothioated nucleotides may be in a second region of the antisense strand, wherein the second region of the antisense strand is 3' of the cassette.

The term *"first region of the sense strand'* as used herein is intended to encompass the part of the sense strand of the linear double-stranded DNA molecule that is between the 5'-end of the linear double-stranded DNA molecule and the first 5' nucleotide of the cassette in the sense strand. For example, in the sequence of the sense strand: 5'-AAAAAACATAAAA-3' (SEQ ID NO: 42), wherein the cassette starts from nucleotide "T" in the 5'-3' direction, the term *"first region of the sense strand'* refers to the 5'-AAAAAACA-3' region.

The term *"first region of the antisense strand'* is intended to encompass the part of the antisense strand of the linear double-stranded DNA molecule that is between the 5'-end of the linear double-stranded DNA molecule and the first 5' nucleotide of the cassette in the antisense strand. For example, in the sequence of the antisense strand: 5'-AAAAAACATAAAA-3' (SEQ ID NO: 42), wherein the cassette starts from nucleotide "T" in the 5'-3' direction, the term *"first region of the antisense strand'* refers to the 5'-AAAAAACA-3' region.

The term *"second region of the sense strand'* is intended to encompass the part of the sense strand of the linear double-stranded DNA molecule that is between the 3'-end of the linear double-stranded DNA molecule and the first 3' nucleotide of the cassette in the sense strand. For example, in the sequence of the sense strand: 5'-AAAAAACATAAAA-3' (SEQ ID NO: 42), wherein the cassette starts from nucleotide "T" in the 3'-5' direction, the term *"second region of the sense strand'* refers to the 5'-AAAA-3' region.

The term *"second region of the antisense strand"* is intended to encompass the part of the antisense strand of the linear double-stranded DNA molecule that is between the 3'-end of the linear double-stranded DNA molecule and the first 3' nucleotide of the cassette in the antisense strand. For example, in the sequence of the antisense strand: 5'-AAAAAACATAAAA-3' (SEQ ID NO: 42), wherein the cassette starts from nucleotide "T" in the 3'-5' direction, the term *"second region of the antisense strand'* refers to the 5'-AAAA-3' region.

The linear double-stranded DNA molecule may comprise a plurality of the phosphorothioated nucleotides upstream (i.e. towards the 5'-end of the sense strand of the DNA molecule) of the cassette. For example, the linear double-stranded DNA molecule may comprise at least 2, 3, 4, 5, 6, 7, 8, 9 or 10 phosphorothioated nucleotides upstream of the cassette. Preferably, at least 2 phosphorothioated nucleotides upstream of the cassette. Thus, the location of phosphorothioated nucleotides in the sense strand of the linear double-stranded DNA molecule may be such that at least two phosphorothioated nucleotides are located upstream of the cassette.

In the sense strand of the linear double-stranded DNA molecule:
(a) one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
(b) the at least two phosphorothioated nucleotides may be in a first region of the sense strand,
wherein the first region of the sense strand is 5' of the cassette.

The linear double-stranded DNA molecule may comprise a plurality of the phosphorothioated nucleotides downstream (i.e. towards the 3-end of the DNA molecule) of the cassette. For example, the linear double-stranded DNA molecule may comprise at least 2, 3, 4, 5, 6, 7, 8, 9 or 10 phosphorothioated nucleotides downstream of the cassette, preferably at least 2 phosphorothioated nucleotides downstream of the cassette. Thus, the location of phosphorothioated nucleotides in the sense strand of the linear double-stranded DNA molecule may be such that at least two phosphorothioated nucleotides are located downstream of the cassette.

In the sense strand of the linear double-stranded DNA molecule:
(a) one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
(b) the at least two phosphorothioated nucleotides may be in a first region of the sense strand, wherein the first region of the sense strand is 3' of the cassette.

The location of phosphorothioated nucleotides in the antisense strand of the linear double-stranded DNA molecule may be such that at least two phosphorothioated nucleotides are located upstream of the cassette (i.e. towards the 5'-end of the antisense strand of the DNA molecule).

In the antisense strand of the linear double-stranded DNA molecule:
(a) one of the at least two phosphorothioated nucleotides is the 5'-end nucleotide of the cassette and one of the at least two phosphorothioated nucleotides is in a first region of the antisense strand, wherein the first region of the antisense strand is 5' of the cassette; or
(b) the at least two phosphorothioated nucleotides are in a first region of the antisense strand, wherein the first region of the antisense strand is 5' of the cassette.

The location of phosphorothioated nucleotides in the antisense strand of the linear double-stranded DNA molecule may be such that at least two phosphorothioated nucleotides are located downstream of the cassette (i.e. towards the 3'-end of the antisense strand of the DNA molecule).

In the antisense strand of the linear double-stranded DNA molecule:
(a) one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
(b) the least two phosphorothioated nucleotides may be in a second region of the antisense strand, wherein the second region of the antisense strand is 3' of the cassette.

Each strand of the linear double-stranded DNA molecule may comprise a plurality of phosphorothioated nucleotides. For example, the linear double-stranded DNA molecule may comprise at least 2, 3, 4, 5, 6, 7, 8, 9 or 10 phosphorothioated nucleotides upstream and downstream of the cassette. The location of phosphorothioated nucleotides in both the sense and the antisense strand of the linear double-stranded DNA molecule may be such that the at least two phosphorothioated nucleotides are located downstream of the cassette and at least two phosphorothioated nucleotides are located upstream of the cassette in each strand.

In the linear double-stranded DNA molecule:
(a) in the sense strand:
   i. one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
   ii. the at least two phosphorothioated nucleotides may be in a first region of the sense strand, wherein the first region of the sense strand is 5' of the cassette;
(b) in the sense strand:
   i. one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
   ii. the at least two phosphorothioated nucleotides may be in a second region of the sense strand, wherein the second region of the sense strand is 3' of the cassette;
(c) in the antisense strand:
   i. one of the at least two phosphorothioated nucleotides may be the 5'-end nucleotide of the cassette and one of the at least two phosphorothioated nucleotides may be in a first region of the antisense strand, wherein the first region of the antisense strand is 5' of the cassette; or
   ii. the at least two phosphorothioated nucleotides may be in a first region of the antisense strand, wherein the first region of the antisense strand is 5' of the cassette; and
(d) in the antisense strand:
   i. one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
   ii. the at least two phosphorothioated nucleotides may be in a second region of the antisense strand, wherein the second region of the antisense strand is 3' of the cassette.

The linear double-stranded DNA molecule or the closed linear DNA molecule (e.g. the covalently closed linear DNA molecule) may be resistant to nuclease digestion or may have improved or enhanced resistance to nuclease digestion. The linear double-stranded DNA molecule or the closed linear DNA molecule may be resistant to exonuclease digestion or have improved or enhanced resistance to exonuclease digestion. The linear double-stranded DNA molecule or the closed linear DNA molecule may be resistant, or have improved or enhanced resistance, to digestion by exonucleases that cleave the 3'-end nucleotides (e.g. exonuclease III) and/or exonucleases that cleave the 5'-end nucleotides (e.g. exonuclease VIII). The terms "improved" or "enhanced" in the context of resistance to enzyme digestion refer to higher resistance to enzyme digestion when compared to a DNA molecule not produced by the methods described herein. For example, for the linear double-stranded DNA molecule, when compared to a molecule that does not comprise protected nucleotides, such as phosphorothioated nucleotides.

The linear DNA molecule (e.g. the linear double-stranded DNA molecule) or the closed linear DNA molecule (e.g. the covalently closed linear DNA molecule) may comprise a functional portion.

The functional portion may be at the 3' and/or the 5' end (or end region) of the polynucleotide chain. The functional portion may be attached or bound to the 5' overhang, the 3' overhang and/or the blunt end of the linear DNA molecule (e.g. the linear double-stranded DNA molecule) or the closed linear DNA molecule. The functional portion may be attached or bound to the 5' overhang, the 3' overhang and/or the blunt end of linear DNA molecule (e.g. the linear double-stranded DNA molecule) or the closed linear DNA molecule by covalent linkage or non-covalent linkage, or by nucleic acid hybridisation. The functional portion may be attached or bound to linear DNA molecule (e.g. the linear double-stranded DNA molecule) or the closed linear DNA molecule directly or indirectly (e.g. via a linker molecule). The functional portion may be attached or bound by being bound to the linear DNA molecule (e.g. the linear double-stranded DNA molecule) or the closed linear DNA molecule and/or by being bound or annealed to linker molecules that are bound to the linear DNA molecule (e.g. the linear double-stranded DNA molecule) or the closed linear DNA molecule. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol).

The DNA molecule may comprise two functional portions; a first functional portion at the 3'-end of the polynucleotide chain, and a second functional portion at the 5'-end of the polypeptide chain. The first functional portion may be attached or bound to the 5' overhang, the 3' overhang and/or the blunt end of the 3'-end of the DNA molecule. The second functional portion may be attached or bound to the 5' overhang, the 3' overhang and/or the blunt end of the 5'-end of the DNA molecule. The two functional portions may be the same or different. For example, the first functional portion may be a barcode to facilitate detection and/or sequencing of the DNA molecule, the second functional portion may be a nuclear targeting sequence.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA molecule by binding to capture molecules (e.g. capture antibodies bound by protein-protein interactions). The functional portion may bind to a cell target, for example, a cell receptor.

The functional portion may be a label. Thus, the sense strand of the DNA molecule may comprise a label at the 5'-end or the 3'-end for detection. Alternatively or additionally, the antisense strand of the DNA molecule may comprise a label at the 5'-end or the 3'-end for detection. The 'label' can be any chemical entity which enable the detection of the double-stranded nucleic acid molecule via, physical, chemical and/or biological means. The label may be a chromophore, a fluorophore and/or a radioactive molecule.

To facilitate detection and/or quantification of the DNA molecule, the functional portion may comprise a fluorophore, a radioactive compound or a barcode.

A signal corresponding to the presence, absence and/or level of the DNA molecule may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the DNA molecule. The binding moiety is capable of binding to 3' and/or 5' end of the DNA molecule. The signal may be measured by determining the presence, absence and/or level of the barcoded portion of the barcode (e.g. by sequencing or PCR). The barcoded portion may comprise at least 2, 3, 4, 5, 6, 7, 8, 9, or 10 nucleotides. The barcode may comprise at least 2 binding moieties (e.g. a first binding moiety and a second binding moiety). For example, the first binding moiety linked to the first barcoded portion may bind to the 3' end of the DNA molecule and the second binding moiety linked to the second barcoded portion may bind to the 5' end of the DNA molecule. The 3' and 5' ends may comprise a 3' overhang, a 5' overhang or a blunt end.

A signal corresponding to the presence, absence and/or level of the DNA molecule may be measured using a fluorophore (i.e. a fluorescently-labelled molecule), which is attached or bound to the 3' overhang, the 5' overhang or the blunt end of the linear molecule. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

### 4. Pharmaceutical compositions

The invention provides a pharmaceutical composition comprising a nanoparticle (e.g. a non-viral transfection complex) described herein and a pharmaceutically suitable carrier or excipient. The invention provides a pharmaceutical composition comprising a targeting peptide described herein and a pharmaceutically suitable carrier or excipient.

The invention provides a pharmaceutical composition comprising:
i. a nanoparticle (e.g. a non-viral transfection complex), which comprises:
   (a) a nucleic acid;
   (b) a lipid component; and
   (c) a targeting peptide comprising a targeting sequence; and
ii. a pharmaceutically suitable carrier.

The pharmaceutical composition may comprise:
i. a nanoparticle (e.g. a non-viral transfection complex), which comprises:
   (a) a nucleic acid
   (b) a lipid component; and
   (c) a targeting peptide comprising a targeting sequence and a nucleic acid-binding component; and
ii. a pharmaceutically suitable carrier.

The pharmaceutical composition may comprise:
i. a nanoparticle (e.g. the non-viral transfection complex), which comprises:
   (a) a nucleic acid;
   (b) a lipid component; and
   (c) a targeting peptide comprising a targeting sequence and a nucleic acid-binding polycationic component (e.g. DNA-binding polycationic component); and
ii. a pharmaceutically suitable carrier.

The pharmaceutical composition may comprise:
i. a nanoparticle (e.g. the non-viral transfection complex), which comprises:
   (a) a closed linear DNA molecule;
   (b) a lipid component; and
   (c) a targeting peptide comprising a targeting sequence and a closed linear DNA-binding component (e.g. a closed linear DNA-binding polycationic component); and
ii. a pharmaceutically suitable carrier.

The pharmaceutical composition may comprise:
i. a nanoparticle (e.g. the non-viral transfection complex) may comprise:
   (a) a linear DNA molecule comprising one or more nuclease-resistant nucleotides (e.g. phosphorothioated nucleotides);
   (b) a lipid component; and
   (c) a targeting peptide comprising a targeting sequence and a linear DNA-binding component (e.g. a linear DNA-binding polycationic component); and
ii. a pharmaceutically suitable carrier.

The pharmaceutical composition may comprise:
i. a targeting peptide comprising:
   (a) a targeting sequence; and
   (b) a nucleic acid-binding component; and
ii. a pharmaceutically suitable carrier.

The pharmaceutical composition may comprise:
i. a targeting peptide comprising:
   (a) a targeting sequence
   (b) a spacer; and
   (c) a nucleic acid-binding component; and
ii. a pharmaceutically suitable carrier.

The pharmaceutical composition may comprise:
i. a targeting peptide comprising:
   (a) a targeting sequence;
   (b) a linker; and
   (c) a nucleic acid-binding component; and
ii. a pharmaceutically suitable carrier.

The pharmaceutical composition may comprise:
i. a targeting peptide comprising:
   (a) a targeting sequence;
   (b) a linker;
   (c) a spacer; and
   (d) a nucleic acid-binding component; and
ii. a pharmaceutically suitable carrier.

The targeting sequence may be any of the targeting sequences described herein. Preferably, the targeting sequence is SEQ ID NO: 1, SEQ ID NO: 6, SEQ ID NO: 7, or SEQ ID NO: 8, or a variant thereof comprising one or more conservative amino acid substitutions.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

The pharmaceutical composition may be administered by injection (e.g. intravenous or intramuscular injection). The pharmaceutical composition may be formulated as an emulsion consisting of a sterile, pyrogen-free preparation or a lipid complex preparation.

For example, the pharmaceutical composition may be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

In another mode of administration, the pharmaceutical composition can be administered in situ, via a catheter or pump. A catheter or pump can, for example, direct the composition into the target location.

The parenteral carrier system (e.g. intravenous carrier system or intramuscular carrier system) may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 5. Uses and applications

### General therapeutic and diagnostic uses

The invention provides the nanoparticle described herein for use in therapy.

The invention provides the targeting peptide described herein for use in therapy.

The invention also provides the pharmaceutical composition described herein for use in therapy.

The nanoparticle may comprise a nucleic acid cargo described herein which encodes a sequence of a therapeutic protein, a part of a vaccine, or an element of a genetic engineering mechanism, which is used to treat a disease or infection in a subject.

The nanoparticle described herein, the targeting peptide described herein, or the pharmaceutical composition described herein may be used in the treatment of a disease or disorder. The disease or disorder may be a genetic disease or disorder (e.g. a monogenic genetic disorder), a disease or disorder of the CNS (such as a neurodegenerative disease (e.g. Parkinson's disease and/or a polyglutamine disease such as Huntington's disease)), a metabolic disease or disorder, a muscle disease or disorder, a disease of disorder of the spleen, or a viral infection (for example, coronavirus (e.g. COVID-19), herpes simplex virus type 2, HIV, influenza, and/or measles).

The disease or disorder may be a muscle disease or disorder, for example, a genetic muscle disease or disorder. The genetic muscle disease may be muscular dystrophy e.g. Duchenne muscular dystrophy, myotonic dystrophy, facioscapulohumeral muscular dystrophy or Becker muscular dystrophy.

Preferably, the nanoparticle described herein, the targeting peptide described herein, or the pharmaceutical composition described herein may be s used to treat a genetic disorder e.g. a monogenic disorder. For example, the nanoparticle described herein, the targeting peptide described herein, or the pharmaceutical composition described herein may be may be used to treat sickle cell anaemia, Huntington disease, Duchenne's Muscular Dystrophy, Haemophilia A, α1-antitrypsin deficiency, or primary ciliary dyskinesia.

The nanoparticle described herein, the targeting peptide described herein, or the pharmaceutical composition described herein may be used as a medicament. The invention provides the nanoparticle described herein, the targeting peptide described herein, or the pharmaceutical composition described herein for use in the manufacture of a medicament for the prophylaxis of a condition caused in a subject by a defect and/or deficiency in a gene.

The invention provides a method for treatment of a disease or disorder caused in a subject by defect and/or deficiency in a gene, the method comprising administering the nanoparticle described herein, the targeting peptide described herein, or the pharmaceutical composition described herein to the subject. Preferably, the amount of the nanoparticle, the targeting peptide or the pharmaceutical composition administered to the subject is a therapeutically active amount. Thus, the invention also provides the nanoparticle described herein, the targeting peptide described herein, or the pharmaceutical composition described herein may be used in gene therapy.

A subject treated with the nanoparticle described herein, the targeting peptide described herein, or the pharmaceutical composition described herein may receive the nanoparticle described herein, the targeting peptide described herein, or the pharmaceutical composition described herein with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the nanoparticle described herein, the targeting peptide described herein, or the pharmaceutical composition described herein into the subject's body as described in more detail above (see "Pharmaceutical compositions"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the nanoparticle described herein, the targeting peptide described herein, or the pharmaceutical composition described herein that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides use of the nanoparticle described herein, the targeting peptide described herein, or the pharmaceutical composition described herein in a method of diagnosing a disease and/or a disorder.

The invention provides the use of the nanoparticle described herein, the targeting peptide described herein, or the pharmaceutical composition described herein in the "in vitro" diagnosis of a disease.

The invention also provides the nanoparticle described herein, the targeting peptide described herein, or the pharmaceutical composition described herein for use in a method of diagnosis "in vivo" of disease.

The nanoparticle described herein, the targeting peptide described herein, or the pharmaceutical composition described herein may be used to diagnose a disease or disorder. The disease or disorder may be a genetic disease or disorder (e.g. a monogenic genetic disorder), a disease or disorder of the CNS (such as a neurodegenerative disease (e.g. Parkinson's disease and/or a polyglutamine disease such as Huntington's disease)), a metabolic disease or disorder, a muscle disease or disorder, a disease of disorder of the spleen, or a viral infection (for example, coronavirus (e.g. COVID-19), herpes simplex virus type 2, HIV, influenza, and/or measles).

The disease or disorder may be a muscle disease or disorder, for example, a genetic muscle disease or disorder. The genetic muscle disease may be muscular dystrophy e.g. Duchenne muscular dystrophy, myotonic dystrophy, facioscapulohumeral muscular dystrophy or Becker muscular dystrophy.

Preferably, the disease or disorder is a genetic disorder e.g. a monogenic disorder. For example, sickle cell anaemia, Huntington disease, Haemophilia A, α1-antitrypsin deficiency or primary ciliary dyskinesia.

The diagnostic and treatment methods described herein may be in vitro methods or in vivo methods.

The method of diagnosis may rely on the detection and/or quantification of the nanoparticle described herein, the targeting peptide described herein, or the pharmaceutical composition described herein.

To facilitate detection and/or quantification of the nanoparticle described herein, the nanoparticle may be attached or bound to a functional portion. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the nanoparticle For example, the signal may be measured by flow cytometry and/or fluorescence-activated cell sorting of the nanoparticle attached to a fluorescent probe.

The nanoparticle may be detected by being bound to a capture moiety, for example in a lateral flow assay. In this example, the functional portion is a protein, for example, an antibody specific for the capture moiety. The capture of the antibody attached to the nanoparticle may produce a visual signal (e.g. a band of a different colour).

The invention also provides a method that combines diagnosis of a disease with a treatment of the disease.

### Cell therapy

The nanoparticle described herein, the targeting peptide described herein, or the pharmaceutical composition described herein may be used in cell therapy.

The invention provides the use of the nanoparticle described herein, the targeting peptide described herein, or the pharmaceutical composition described herein in the production of cell therapy. The invention provides a method of cell therapy, comprising contacting the nanoparticle described herein, the targeting peptide described herein, or the pharmaceutical composition described herein with a cell. Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

### Vaccines

The products of the invention are particularly suitable for use in vaccine production. The nanoparticle described herein, the targeting peptide described herein, or the pharmaceutical composition described herein may be used to produce a vaccine, preferably an mRNA-based vaccine. For example, vaccines such as the BioNTech and Moderna mRNA vaccines against COVID-19.

The nanoparticle described herein or the pharmaceutical composition described herein may be used as a vaccine.

The nanoparticle described herein, the targeting peptide described herein, or the pharmaceutical composition described herein may be used in the therapeutic or prophylactic immunisation.

### CRISPR delivery

The nanoparticles and targeting peptides are particularly suitable for use in delivery of CRISPR machinery to cells, for example in cell therapy or in vivo therapy.

The nanoparticle may comprise a cargo, which is a nucleic acid cargo (e.g. the nucleic acid cargo described herein). The nucleic acid cargo may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). The repair template (or editing template) may comprise at least 10, at least 20, at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, 10000, at least 11000, at least 12000, at least 13000, at least 14000, or at least 15000 base pairs.

Thus, the invention provides the nanoparticle for use in CRISPR system delivery to a cell. The invention provides a method of delivering the CRISPR system to a cell, comprising contacting the nanoparticle with a cell.

The cell may be an animal cell, preferably mammal cell, such as human cell.

The invention also provides a cell obtainable by the methods described herein. The cell is particularly suitable for use in cell therapy and/or in vivo therapy.

Uses of the products described herein may be *in vivo* or *in vitro* uses.

### 6. Methods for producing nanoparticles

The invention provides a method for producing (or forming) a nanoparticle described herein, wherein the method comprises:
(a) contacting a lipid component with a closed linear DNA molecule and a targeting peptide comprising a targeting sequence to form a single contiguous aqueous volume; and
(b) forming the nanoparticle.

The invention also provides a method for producing (or forming) a nanoparticle described herein, wherein the method comprises:
(a) contacting a lipid component with a linear DNA molecule comprising one or more protected nucleotides and a targeting peptide comprising a targeting sequence to form a single contiguous aqueous volume; and
(b) forming the nanoparticle.

The invention provides a method for producing (or forming) a nanoparticle described herein, wherein the method comprises:
(a) contacting a lipid component with a closed linear DNA molecule and a targeting peptide comprising a targeting sequence; and
(b) forming the nanoparticle.

The invention also provides a method for producing (or forming) a nanoparticle described herein, wherein the method comprises:
(a) contacting a lipid component with a linear DNA molecule comprising one or more protected nucleotides and a targeting peptide comprising a targeting sequence; and
(b) forming the nanoparticle.

The lipid component may be a liposome. The method may comprise the steps:
(a) contacting a liposome with a closed linear DNA molecule and a targeting peptide; and
(b) forming the nanoparticle.

The method may comprise the steps:
(a) contacting a liposome with a linear DNA molecule comprising one or more protected nucleotides and a targeting peptide; and
(b) forming the nanoparticle.

The method may further comprise a step of forming a liposome. The step of forming a liposome may be performed before the step of contacting a liposome with a closed linear DNA molecule (or a linear DNA molecule comprising one or more protected nucleotides) and a targeting peptide.

The step of forming the nanoparticle may be performed by shaking, pipetting or using a microfluidics device.

The method may further comprise a step of diluting the nanoparticle to a desired nucleic acid concentration. The step of diluting the nanoparticle to a desired nucleic acid concentration after the step of forming the nanoparticle. For example, if the nucleic acid is a closed linear DNA molecule, the desired nucleic acid concentration may be 0.01 ng/µL - 10,000 ng/µL, 0.1 ng/µL - 1000 ng/µL or 1 ng/µL - 100 ng/µL.

### 7. Cell transfection

The invention provides a cell transfection composition comprising the nanoparticle described herein.

The invention provides a method for transfecting a cell comprising:
(a) contacting a cell with the nanoparticle described herein; and
(b) transfecting the nanoparticle to the cell.

The cell may be an animal cell, such as mammal cell (e.g. a human cell), a fungal cell, a cell of a micro-organism (e.g. a prokaryotic cell or a eukaryotic cell), or a plant cell. The cell may be a human cell. The cell may be a C2C12 cell or a HEK293 cell.

The cell transfection composition may or may not comprise an agent selected from a photosensitizing agent and/or a radical initiator e.g. a photoinitiator. Preferably, this agent improves the function of the cargo (e.g. the nucleic acid cargo described herein) at a target site and/or protects the cargo (e.g. the nucleic acid cargo described herein) from metabolism and/or degradation.

The invention further provides a cell obtainable by the methods of the invention. Thus, the cell may comprise the nanoparticle of the invention.

The invention further provides a cell transfected with the nanoparticle described herein.

The step of contacting the cell with the nanoparticle may be performed in vivo. For example, the nanoparticle may be administered to an organism (e.g. a subject) in need thereof. The organism (e.g. the subject) may be an animal, such as mammal (e.g. a human), a fungus, a micro-organism, or a plant.

### BRIEF DESCRIPTION OF THE DRAWINGS

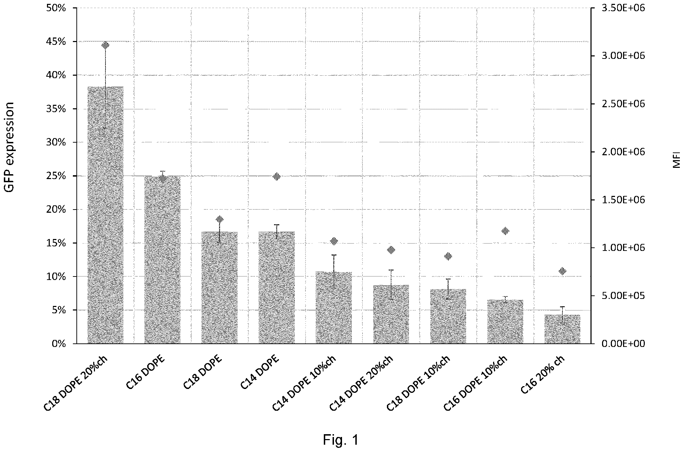

Figure 1 shows GFP expression and median fluorescence intensity of HEK293 cells transfected with hpDNA-lipid-peptide nanoparticles containing different cationic lipids with varying alkyl chain lengths, and different molar percentages of cholesterol and a peptide with the amino acid sequence K16GACYGLPHKFC (Peptide Y). GFP expression was measured 48h post transfection. n=3 in all experiments, error bars = S.D. "Ch" is an abbreviation of cholesterol.

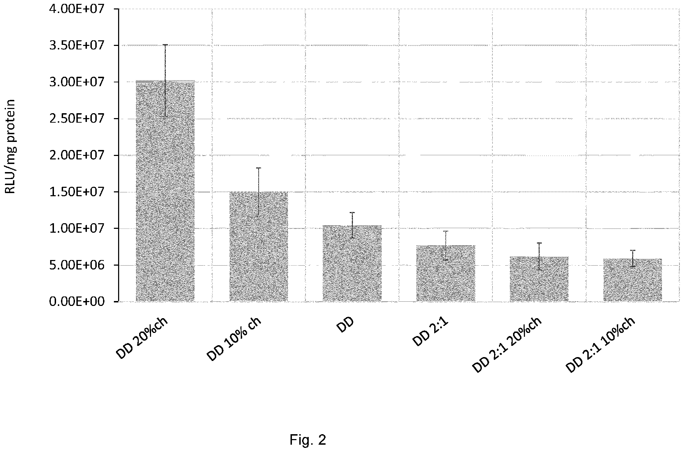

Figure 2 shows Luciferase expression in HEK293 cells transfected with hpDNA-lipid-peptide nanoparticles containing different molar ratios of the cationic lipid DOTMA, the phospholipid DOPE, and various molar percentages of cholesterol, and a peptide with the amino acid sequence K16GACYGLPHKFC. Luciferase expression was measured 48h post transfection. n=3 in all experiments, error bars = S.D. "DD" stands for an equimolar ratio of DOTMA:DOPE, while "DD 2:1" stands for a 2:1 molar ratio of DOTMA:DOPE. "Ch" is an abbreviation of cholesterol.

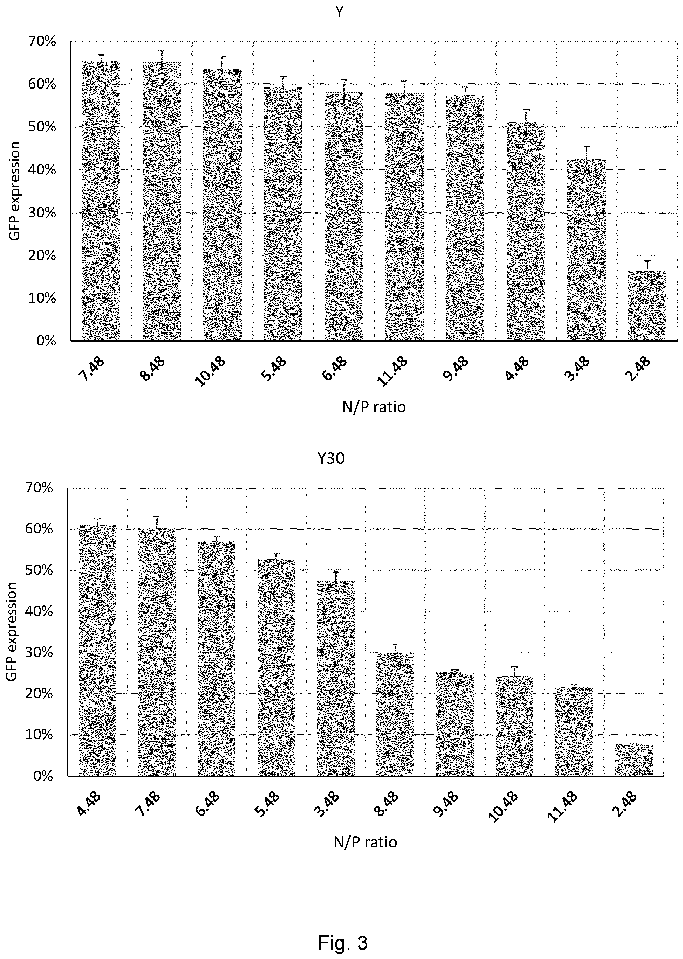

Figure 3 shows GFP expression and median fluorescence intensity of HEK293 cells transfected with hpDNA-lipid-peptide nanoparticles containing DOTMA DOPE with 20% cholesterol, and varying amounts of peptides with the amino acid sequences K16GACYGLPHKFC (Peptide Y) and K30GACYGLPHKFC (Peptide Y30), driving the N/P ratio of the nanoparticle. GFP expression was measured 48h post transfection. n=3 in all experiments, error bars = S.D.

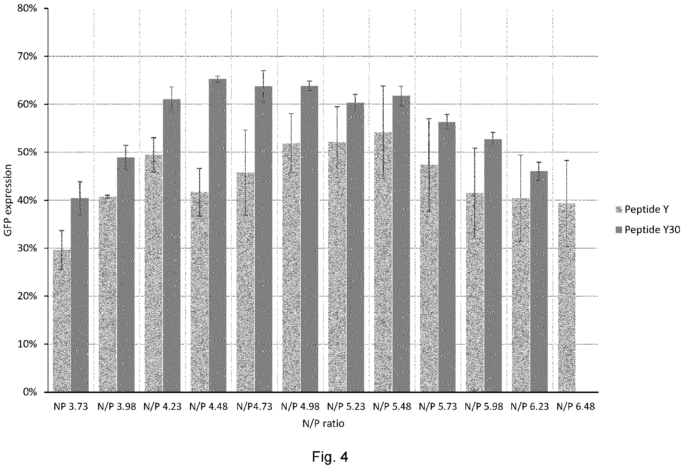

Figure 4 shows GFP expression and media fluorescence intensity of HEK293 cells transfected with hpDNA-lipid-peptide nanoparticles containing different DOTMA DOPE with 20% cholesterol, and varying amounts of peptides with the amino acid sequences K16GACYGLPHKFC (Peptide Y) and K30GACYGLPHKFC (Peptide Y30), driving the N/P ratio of the nanoparticle. GFP expression was measured 48h post transfection. n=3 in all experiments, error bars = S.D.

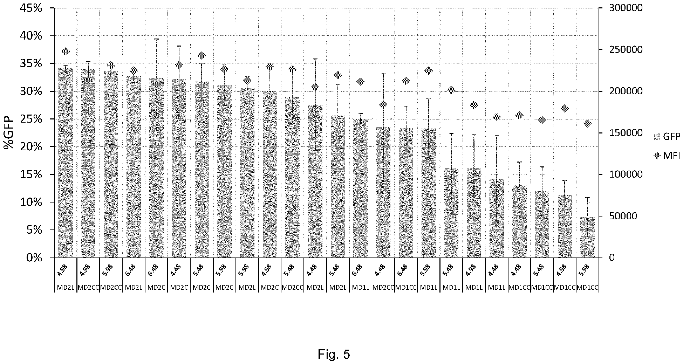

Figure 5 shows GFP expression in C2C12 cells transfected with hpDNA-lipid-peptide nanoparticles containing peptides with different targeting ligands specific for muscle cells, with different structural conformations of the targeting ligand. A range of lipids with varying molar ratios of DOTMA, DOPE and cholesterol were explored. GFP expression was measured 48h post transfection. n=3 in all experiments, error bars = S.D.

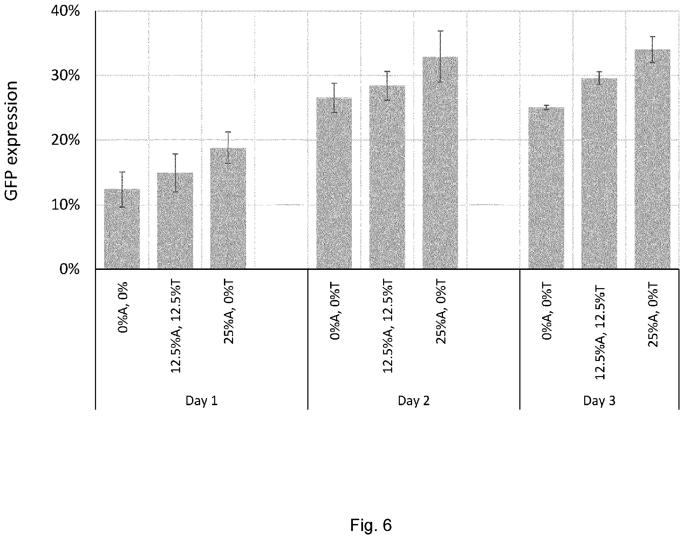

Figure 6 shows GFP expression in HEK293 cells transfected with open linear DNA-lipid-peptide nanoparticles containing DD 10% cholesterol and Peptide Y. 3 open linear DNA constructs were tested: no protection, 12.5% A and 12.5% T protection, and 25% A protection. GFP expression was measure at 24, 48 and 72 hours. n=3 in all experiments, error bars = S.D.

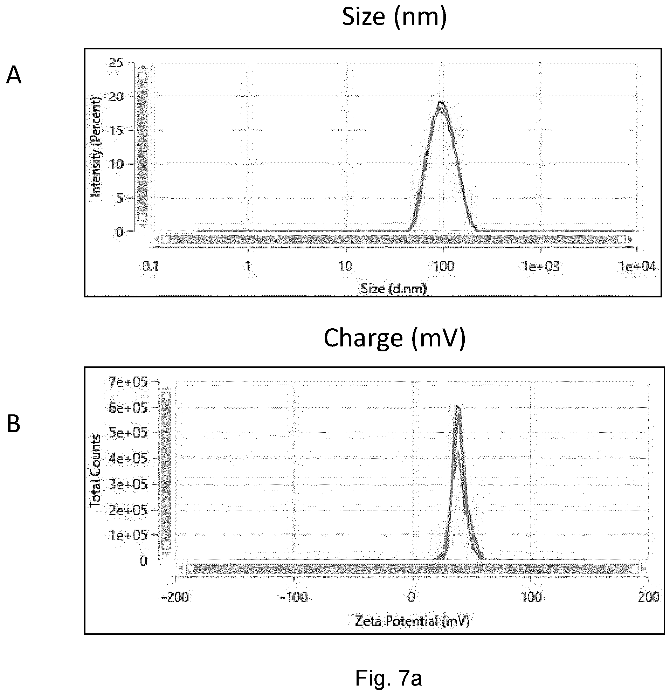

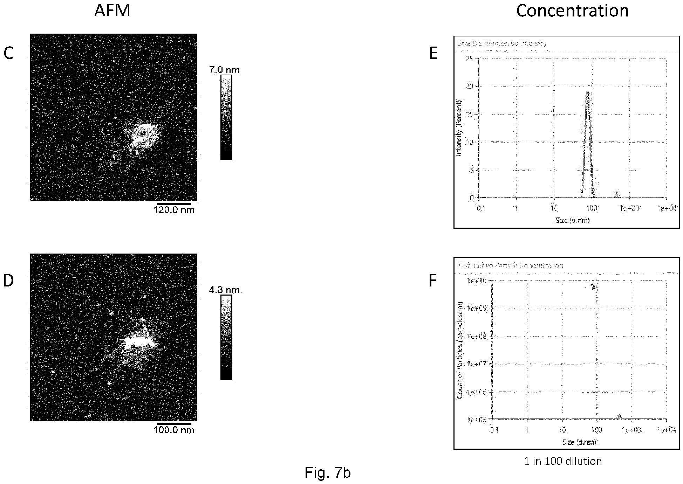

Figure 7 (7a and 7b) shows the biophysical characterisation of a representative hpDNA-lipid-peptide nanoparticle containing C18DOPE 10% cholesterol and Peptide Y (SEQ ID NO: 4). A shows size of the particles in nm by Dynamic Light Scattering (DLS). B shows the zeta potential of the nanoparticle in mV Electrophoretic Light Scattering (ELS). C and D show representative atomic force microscopy images of nanoparticles. E and F show the concentration of the nanoparticles by Multi-Angle Dynamic Light Scattering (MADLS).

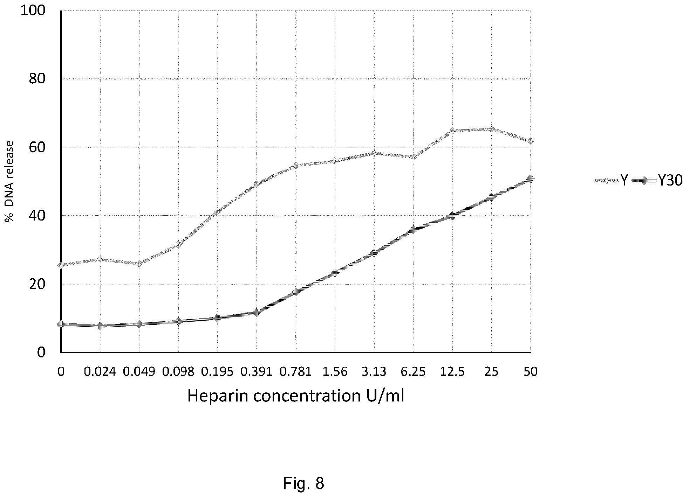

Figure 8 shows the stability and dissociation properties of hpDNA-lipid-peptide nanoparticles containing different DOTMA DOPE with 20% cholesterol and peptides with the amino acid sequences K16GACYGLPHKFC (Peptide Y) or K30GACYGLPHKFC (Peptide Y30). hpDNA was incubated with Picogreen fluorescent nucleic acid stain, prior to nanoparticle formulation. Nanoparticles were incubated with heparin (0.02-5 U/ml) and percentage hpDNA release was calculated using free hpDNA. n=3 in all experiments.

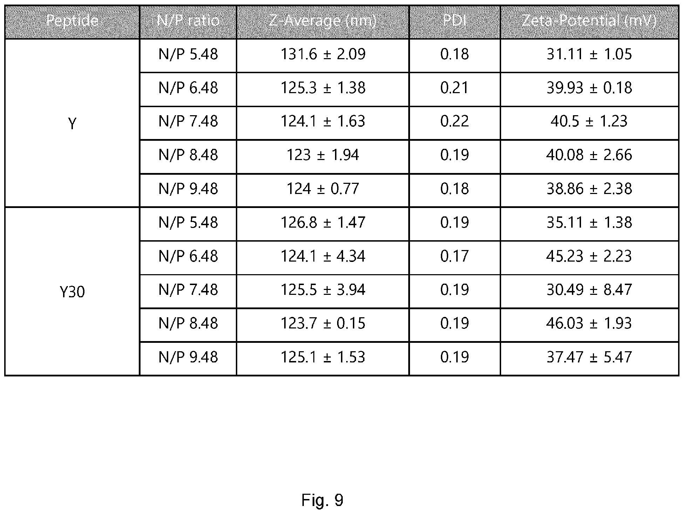

Figure 9 shows the biophysical characterisation of hpDNA-lipid-peptide nanoparticle containing DD 10% cholesterol with either Peptide Y or Y30, at various N/P ratios driven by the mass of peptide. Size of the particles in nm, polydispersity of the particles, and zeta potential in mV are included.

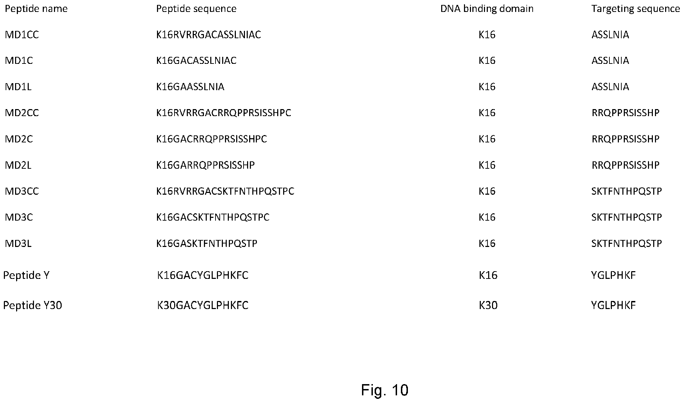

Figure 10 provides details of exemplary targeting peptides.

### EXAMPLES

### Example 1 - Liposome formulation

Liposomes were made using NanoAssemblr Ignite, a microfluidcs device (Precison Nanosystems, Vancouver Canada). The cationic lipid and phospholipid/cholesterol were mixed together at various molar ratios in ethanol and injected into the cartridge at a flow rate of 12mL/ min. They were then sonicated in a water bath for 20 minutes. The resulting liposomes were dialysed overnight in 10k Slide-A-Lyzer (Thermo Fisher) cassettes to remove any residual ethanol. Prior to use in experiments, liposomes were stored at 4°C.

### Example 2- Luciferase assay

Peptides K16GACYGLPHKFC (Peptide Y, SEQ ID NO: 4) and K30GACYGLPHKFC (Peptide Y30, SEQ ID NO: 5) were synthesised via solid-phase peptide synthetic chemistry. DOTMA and DOPE was purchased from Avanti Polar Lipids, Alabaster, Alabama, USA. Cholesterol was purchased from Sigma Aldrich, UK.

HEK293 cells, an immortalised human embryonic kidney were cultured in Dulbecco's Modified Eagle's Medium (DMEM, Gibco) containing 10% fetal bovine serum (FBS, Gibco) and 1% pen/strep (Gibco).

Transfections in HEK293 cells were performed with a range of peptide: liposome: Luciferase hpDNA ratios. Transfections were performed in a 96-well plate at a density of 16,000 cells per well seeded 24 h previously. Charge and mass ratio of components in the transfection complex varied; hpDNA was maintained at a constant mass of 300 ng per well, while lipid component was altered. In one case, the molar ratio of the cationic lipid C18 to the phospholipid DOPE was kept at 1:1. In another case, the molar ratio of the cationic lipid C18 to the phospholipid DOPE was kept at 2:1, while cholesterol was added at a molar percentage of 10 or 20% of the total lipid content The mass ratio of hpDNA:liposome:peptide was maintained at 1:3:3.

Transfection complexes were formed via electrostatic interaction by vigorous pipetting, before diluting in a final volume of 200 ul per well, with a DNA concentration of 1.5ng/ul. All dilutions were performed with OptiMEM reduced serum media. Controls included cells with no transfection complexes added (OptiMEM only).

Transfection complexes were incubated for 30 min at RT before adding to cells. All conditions were performed in triplicate. Cells were incubated at 37°C for 4 hours, before replacing media with growth media. 48 h post-transfection, the cells were rinsed in PBS, before resuspension in Reporter Lysis buffer (Promega). Plates were incubated at 4°C for 20 minutes, before -80°C for 40 minutes. After thawing, luciferase activity was measured following injection of the luciferase assay substrate on the ClarioSTAR plus plate reader (BMG Labtech, Aylesbury, UK). Luciferase expression was normalised to protein content using Pierce BCA Protein Assay, with absorbance measured at 562nm. Luciferase activity was expressed as Relative Light Units per mg of protein (RLU/mg).

Figure 2 shows Luciferase expression in RLU/mg protein in HEK293 cells transfected with hpDNA:lipid:peptide nanoparticles comprising different mass ratios of lipid and peptide components. DOTMA/DOPE with 20% cholesterol + Peptide Y (SEQ ID NO: 4) showed highest RLU value (3E+07), indicating greatest transfection efficiency of these nanoparticles. DOTMA/DOPE with 10% cholesterol + Peptide Y, followed by DOTMA/DOPE + Peptide Y with 0% cholesterol showed next highest RLU values. Nanoparticles containing DOTMA/DOPE at a 2:1 molar ratio, with and without cholesterol, showed lowest RLU values of Luciferase expression, indicating lowest transfection efficiencies.

### Example 3 - hpGFP transfection

Peptides K16GACYGLPHKFC (Peptide Y, SEQ ID NO: 4) and K30GACYGLPHKFC (Peptide Y30, SEQ ID NO: 5) were synthesised via solid-phase peptide synthetic chemistry. DOTMA and DOPE was purchased from Avanti Polar Lipids, Alabaster, Alabama, USA. Cholesterol was purchased from Sigma Aldrich, UK.

HEK293 cells, an immortalised human embryonic kidney were cultured in Dulbecco's Modified Eagle's Medium (DMEM, Gibco) containing 10% fetal bovine serum (FBS, Gibco) and 1% pen/strep (Gibco).

Transfections in HEK293 cells were performed with a range of peptide: liposome: GFP hpDNA ratios. Transfections were performed in a 96-well plate at a density of 16,000 cells per well seeded 24 h previously. Charge and mass ratio of components in the transfection complex varied; hpDNA was maintained at a constant mass of 300 ng per well, while lipid component and peptide component were altered.

Transfection complexes were formed via electrostatic interaction by vigorous pipetting, before diluting in a final volume of 200 ul per well, with a DNA concentration of 1.5ng/ul. All dilutions were performed with OptiMEM reduced serum media. Controls included cells with no transfection complexes added (OptiMEM only).

Transfection complexes were incubated for 30 min at room temperature (∼22°C) before adding to cells. All conditions were performed in triplicate. Cells were incubated at 37°C for 4 hours, before replacing media with growth media. 48 h post-transfection, the cells were rinsed in PBS, before incubation in 0.05% Trypsin EDTA (Gibco) to detach the cells. Cells were then resuspended in PBS for analysis by flow cytometry using the Aligent Novocyte flow cytometer.

Figure 1 shows GFP expression in HEK293 cells transfected with hpDNA:lipid:peptide nanoparticles comprising different mass ratios of lipid and peptide components. DOTMA/DOPE with 20% cholesterol + Peptide Y (SEQ ID NO: 4) showed highest GFP expression, with 38% of cells expression GFP with a median fluorescent intensity (MFI) of 3.1E+06, indicating greatest transfection efficiency of these nanoparticles. C16 DOPE, C18 DOPE and C14 DOPE + Peptide Y with 0% cholesterol content showed GFP expression ranging from 24.98% to 16.65%, with MFI values ranging from 1.72E+06 to 1.30E+06. The lowest value of GFP expression was 4.31%, and an MFI of 7.56E+05, using nanoparticles containing C16 DOPE + 20% cholesterol + Peptide Y.

Figure 3 shows GFP expression in HEK293 cells transfected with hpDNA:lipid:peptide nanoparticles containing DD 20% cholesterol with either Peptide Y or Y30 at various N/P ratios driven by the mass of peptide. Transfection efficiencies from the nanoparticles with varying N/P ratios were ranked. For nanoparticles containing Peptide Y, N/P ratios of 7.48, 8.48 and 10.48 performed best, with GFP expression ranging from 65.42% to 63.50%. Lowest N/P ratios, 4.48, 3.48 and 2.48 achieved the lowest GFP expression, ranging from 51.16% for N/P 4.48, to 16.43% with N/P 2.48. For nanoparticles containing Peptide Y30, N/P ratios of 4.48, 7.48 and 6.48 performed best, with GFP expression ranging from 60.83% to 56.99%. Highest N/P ratios, 9.48, 10.48, and 11.48 achieved lower GFP expression, ranging from 25.18% to 21.66%. As with nanoparticles containing Peptide Y, N/P ratio of 2.48 achieved the lowest transfection efficiency- with 7.78% GFP expression. This data suggests that with Peptide Y30, lower N/P ratios, and therefore lower mass of peptide, are required to achieve high GFP expression from hpDNA constructs, which could impart lower toxicity within the cells.

Figure 4 shows GFP expression in HEK293 cells transfected with hpDNA:lipid:peptide nanoparticles containing DD 20% cholesterol with either Peptide Y or Y30 at various N/P ratios driven by the mass of peptide. N/P ratios ranged from 3.73 to 6.48, based on the highest transfection efficiency achieved in Figure 3 by nanoparticle containing Peptide Y30 at an N/P ratio of 4.48. At these lower N/P ratios, nanoparticles containing Peptide Y30 consistently outperformed nanoparticles containing Peptide Y. The highest transfection efficiency achieved was with Peptide Y30 nanoparticles at an N/P ratio of 4.48, with GFP expression of 65.26%, as compared to 41.67% achieved with Peptide Y nanoparticles at the same N/P ratio. The next best performing Peptide Y30 nanoparticles had an N/P ratio of 4.73 and 4.98, with transfection efficiencies of 63.82% and 63.82%, as compared to Peptide Y nanoparticles with the same N/P ratio achieving 45.72% and 51.90% GFP expression respectively. The data series confirms that nanoparticles containing Peptide Y30 require a lower N/P ratio to achieve high transfection efficiencies with hpDNA.

Figure 5 shows GFP expression in C2C12 cells transfected with hpDNA:lipid:peptide nanoparticles DD 2:1 + 10% cholesterol with peptide sequences each identified to bind to muscle-cells, at varying N/P ratios. Nanoparticles containing peptide MD2L at an N/P ratio of 4.98 showed highest GFP expression, with 34% of cells expressing GFP, and an MFI of 2.48E +05, closely followed by MD2CC at an N/P of 4.98, with a transfection efficiency of 33.89%, and an MFI of 2.13E +05. Nanoparticles containing peptide MD1CC achieved the lowest transfection efficiencies across a range of N/P ratios, with GFP expression between 7.26% - 13.09%.

Figure 6 shows GFP expression in HEK293 cells transfected with open linear DNA-lipid-peptide nanoparticles containing DD 10% cholesterol and Peptide Y. 3 open linear DNA constructs were tested: no protection, 12.5% phosphorothioated A and 12.5% phosphorothioated T protection, and 25% phosphorothioated A protection. GFP expression was measure at 24, 48 and 72 hours. Over the course of the experiment, protected constructs consistently performed better than the construct with 0% protection. After 24 h, GFP expression from nanoparticles containing open linear DNA construct with 0% protection achieved a transfection efficiency of 12.35%, as compared to 14.89% and 18.79% for constructs with 12.5% A and 12.5% T protection, and 25% A protection respectively. On day 3, cells transfected with nanoparticles containing open linear DNA construct with 0% protection had 25.08% GFP positive cells compared to 29.59% and 34.02% for constructs with 12.5% A and 12.5% T protection, and 25% A protection respectively.

### Example 4 - Heparin dissociation assay

0.2 µg hpDNA was mixed with 1.33 µL PicoGreen (Thermo Fisher Scientific) (150 ng:1 µL ratio) in TE buffer (10 mM Tris-HCI pH 8, 1 mM EDTA) in 100 µL per well, and were incubated in the dark for 10 minutes at RT. The PicoGreen-labelled hpDNA was then mixed with 0.6 µg DD10% and either Peptide Y (SEQ ID NO: 4) or Y30 (SEQ ID NO: 5), at an overall N/P ratio of 4.48 in 100 µL TE buffer in clear 96 well plate. The nanocomplexes were incubated in the dark for 30 minutes at RT. Several conditions of heparin were prepared, ranging from 0.02 to 50 U/ml. 100 µL Heparin in TE buffer was added in 200 µL nanoparticles. They were incubated in the dark for 30 minutes at RT. Fluorescence intensity (Excitation: 492mm, Emission 520) was measured using the CLARIOstar Optima plate leader. PicoGreen signal detected from the complexes was expressed as percentage relative fluorescence units (RFU). Naked DNA complexed with Picogreen was used as a positive control to normalise the signal. Negative controls included DNA-free nanoparticles.

Figure 8 shows a heparin dissociation assay with nanoparticles formulated with Peptide Y (SEQ ID NO: 4) and Y30 (SEQ ID NO: 5), along with DD 10% cholesterol. Heparin is a highly anionic species, and heparin sulphate proteoglycan is present in the cell surface of mammalian cells. The highly negative sugar is capable of displacing the bound nucleic acid from its cationic vehicle. In this experiment, the DNA is complexed with Picogreen, a fluorescent dye that binds to DNA. When DNA-Picogreen are complexed in particles, the fluorescent signal is quenched. Dissociation of the nanoparticle in the presence of heparin restores the fluorescent signal. With nanoparticles comprised of Peptide Y (SEQ ID NO: 4), the initial encapsulation of DNA is lower than with Peptide Y30 (SEQ ID NO: 5), with an encapsulation efficiency of 74.7%, as compared to 91.9% with Peptide Y30. The particle then begins to dissociate at a heparin concentration of 0.098 U/ml. In increasing concentrations of heparin, the stability of particles containing Peptide Y30 remains stable up until a heparin concentration of 0.391 U/ml, where and increase in fluorescent signal, and therefore DNA release, is detectable. Even at a heparin concentration of 50 U/ml, the DNA is not fully released from either nanoparticle.

### Example 5 - Biophysical characterisation of hpDNA nanoparticles

The size in nm by Dynamic Light Scattering (DLS), zeta potential in mV by Electrophoretic Light Scattering (ELS), and concentration of particles per ml by Multi-Angle Dynamic Light Scattering (MADLS) of the hpDNA-lipid-peptide nanoparticles was determined using the Zetasizer Ultra (Malvern). Complexes were prepared as before, except formulated in nuclease-free water rather than OptiMEM. Samples containing 0.7 µg hpDNA were diluted in 1ml for analysis. The results show that the hpDNA nanoparticles have favourable biophysical characteristics.

Figure 7 shows the biophysical characterisation of a representative hpDNA-lipid-peptide nanoparticle containing C18DOPE 10% cholesterol and Peptide Y, at a weight ratio of 1:3:3 hpDNA:lipid:peptide. A shows size of the particle to be 121 nm. B shows the particle has a zeta potential of 35.11 mV. C and D show representative atomic force microscopy images of the nanoparticle, with a dense, nucleic acid core, and peptides visible encapsulating the particle. E and F show the concentration of the nanoparticle, with a particle titre of 10 E12 particles per ml, accounting for the 1 in 100 dilution.

Figure 9

## Claims

1. A nanoparticle comprising:
(a) a closed linear deoxyribonucleic acid (DNA) molecule;
(b) a lipid component; and
(c) a targeting peptide comprising a targeting sequence.

2. A nanoparticle comprising:
(a) a linear deoxyribonucleic acid (DNA) molecule comprising one or more nuclease-resistance nucleotides;
(b) a lipid component; and
(c) a targeting peptide comprising a targeting sequence.

3. The nanoparticle of claim 1 or claim 2, wherein the nanoparticle is a non-viral transfection complex.

4. The nanoparticle of any one of claims 1-3, wherein the nanoparticle further comprises a nucleic acid-binding component, optionally wherein the nucleic acid-binding component is a nucleic acid-binding cationic component.

5. The nanoparticle of any one of claims 1-4, wherein the targeting sequence targets a cell-surface receptor, optionally wherein the targeting sequence targets a spleen cell, a kidney cell, a muscle cell, a brain cell or a skin cell (i.e. an epithelial cell).

6. The nanoparticle of any one of claims 1-5, wherein the targeting sequence is: SEQ ID NO: 1, SEQ ID NO: 6, SEQ ID NO: 7, or SEQ ID NO: 8 or a variant thereof comprising one or more conservative amino acid substitutions.

7. The nanoparticle of any one of claims 1-5, wherein the targeting peptide comprises: SEQ ID NO: 2, SEQ ID NO: 3, SEQ ID NO: 4, SEQ ID NO: 5, or any one of SEQ ID NO: 9-38, or a variant thereof comprising one or more conservative amino acid substitutions;

8. The nanoparticle of any one of claims 1-7, wherein the charge ratio (i.e. Nitrogen/Phosphate ratio) of the nanoparticle s 2.0-12.0 or 4.0-12.0.

9. The nanoparticle of any one of claims 4-8, wherein the nucleic acid-binding component comprises 16 lysine residues and the negative/positive ratio of the nanoparticle is:
(a) 3.0-12.0 or 4.0-12.0, preferably 7.0-11.0; or
(b) about 7.5, about 8.5, or about 10.5.

10. The nanoparticle of any one of claims 4-8, wherein the nucleic acid-binding component comprises 30 lysine residues and the negative/positive ratio of the nanoparticle is:
(a) 3.0-8.0 or 4.0-8.0; or
(b) about 4.2, about 4.5, about 4.7, about 5.2, about 5.5, about 5.7 about 6.5, or about 7.5.

11. A pharmaceutical composition comprising a nanoparticle of any one of claims 1-10 and a pharmaceutically suitable carrier.

12. The nanoparticle of any one of claims 1-10 or the pharmaceutical composition of claim 11 for use in therapy.

13. The nanoparticle of any one of claims 1-10, or the pharmaceutical composition of claim 11, for use in the treatment of a genetic disease or disorder, a disease or disorder of the CNS, a metabolic disease or disorder, a muscle disease or disorder, a disease of disorder of the spleen, or a viral infection.

14. A method for transfecting a cell comprising:
(a) contacting a cell with the nanoparticle of any one of claims 1-10 or the pharmaceutical composition of claim 11; and
(b) transfecting the nanoparticle or the pharmaceutical composition to the cell.

15. A method for forming the nanoparticle of any one of claims 1-10, the method comprising:
(a) contacting a lipid component with (i) a closed linear DNA molecule or a linear DNA molecule comprising one or more protected nucleotides, and (ii) a peptide comprising a targeting sequence to form a single contiguous aqueous volume; and
(b) forming the nanoparticle.
